# EUROPEAN PATENT APPLICATION

(11) **EP 4 101 465 A1**
(43) Date of publication of application: **14.12.2022**
(21) Application number: 21750738.3
(22) Date of filing: 04.02.2021
(51) Int. Cl.: A61K 45/00, A61P 25/00, A61P 25/16, A61P 25/28, C07K 14/565, C07K 16/18, C07K 16/28, C12N 15/12, C12N 15/13, C12N 9/12, C12N 9/16, C12N 9/99, G01N 33/15, G01N 33/50, G01N 33/53, A61K 31/13, A61K 31/27, A61K 31/445, A61K 31/473, A61K 31/55

(54) **DETERMINATION AGENT AND DETERMINATION METHOD FOR TAUOPATHY AND DEMENTIA-RELATED DISEASES**

(30) Priority: 05.02.2020 JP 2020018249
(71) Applicant: Sumitomo Pharma Co., Ltd., Osaka 541-0045 (JP)
(72) Inventor: ONO, Atsushi, Osaka-shi, Osaka 554-0022 (JP); NAGATA, Hidetaka, Osaka-shi, Osaka 554-0022 (JP); HASHIMOTO, Masakazu, Osaka-shi, Osaka 554-0022 (JP)
(74) Representative: J A Kemp LLP
(86) International application number: PCT/JP2021/004018
(87) International publication number: WO 2021/157634

(57) **Abstract**

The present invention provides a kit for use in determining tauopathy or a dementia-related disease (excluding Alzheimer's disease), containing an antibody that recognizes a polypeptide consisting of (1) the amino acid sequence shown in SEQ ID NO: 1, or (2) an amino acid sequence resulting from substitution, deletion, addition or insertion of one to several amino acids in the amino acid sequence shown in SEQ ID NO: 1, and the like.

## Description

### [Technical Field]

The present invention relates to an agent for determining tauopathy and dementia-related diseases, a method for determining tauopathy and dementia-related diseases, a method for treating tauopathy and dementia-related diseases, and a method for selecting a candidate substance for a therapeutic drug for tauopathy and dementia-related diseases.

### [Background Art]

Tauopathy is a general term for a group of neurodegenerative diseases showing, as a pathological image of the brain, neurofibrillary changes accompanied by abnormal lesions of tau protein, and Alzheimer's disease (hereinafter sometimes referred to as "AD"), progressive supranuclear palsy (hereinafter sometimes referred to as "PSP"), corticobasal degeneration (hereinafter sometimes referred to as "CBD"), multiple system atrophy (hereinafter sometimes referred to as "MSA"), pick disease (hereinafter sometimes referred to as "PiD"), and the like are known.

Dementia is one type of cognitive disorder, and refers to a condition in which the ability to recognize, remember, or judge is disordered by an acquired organic disorder of the brain, which causes difficulty in social life. As future estimates of the number of dementia patients and the prevalence of elderly people aged 65 and over in Japan, the number of dementia patients was 4.62 million in 2012, corresponding to one in seven elderly people aged 65 and over, and the number is expected to reach about 7 million in 2025, corresponding to one in five people. As the four major dementias, Alzheimer's disease, frontotemporal dementia (hereinafter sometimes referred to as "FTD"), dementia with Lewy Bodies (hereinafter sometimes referred to as "DLB"), and vascular dementia (hereinafter sometimes referred to as "VaD") are known, and these are said to account for about 90% of the entire dementia. In addition, there are many cognitive dysfunctions expressed in association with neurodegenerative diseases, such as cognitive dysfunction associated with Parkinson's disease (hereinafter sometimes referred to as "PDD") and multiple sclerosis (hereinafter sometimes referred to as "MS").

PSP is a disease that develops after middle age, in which nerve cells in brain regions such as pallidum, subthalamic nucleus, substantia nigra, and brain-stem tegmentum are lost, and abnormal phosphorylated tau protein accumulates in nerve cells and glial cells. The cause of the onset is unknown, and PSP is often developed in men. The initial symptoms are similar to those of Parkinson's disease, but resting tremor is rare, and easy falling down during walking, freezing of gait, difficulties in keeping postures are prominent. As PSP progresses, retroflexion of cervical part and warped posture, dysarthria and dysphagia, and cognitive dysfunctions and decreased attention characterized by disorder in recollection and slow cerebration appear, followed by gradual occurrence of abasia, difficulty in keeping standing posture, and being bedridden. Even though symptoms may be temporarily improved with antidepressants and droxidopa, the response to anti-Parkinsonian drugs is poor, and no curative treatment has been found to date.

CBD is a disease in which nerve cells in cerebral cortex and subcortical nerve nuclei, particularly substantia nigra and pallidum, are lost and abnormally phosphorylated tau protein accumulates in neurons and glia cells. Typically, CBD develops after middle age and limb-kinetic apraxia, ideomotor apraxia, cortical sensory disorder, grasp reaction, alien hand sign, and the like appear. In addition, akinesia, muscle stiffness, dystonia, and myoclonus appear as extrapyramidal signs, and these neurological symptoms show a remarkable bilateral difference. On the other hand, many atypical cases have been reported such as cases with no bilateral difference, cases with cognitive dysfunction and aphasia in the foreground and cases with clinical symptoms of PSP, and it has been clarified that the clinical feature of CBD is extremely diverse. The etiology is unknown, and familial cases have been reported but rare. There is no curative therapy, and all are treated with symptomatic treatments as the situation stands.

MSA is a disease that develops in adulthood, mostly after the age of 40. It is histologically a disease in which insoluble inclusion bodies of α-synuclein protein accumulate in nerve cells and oligodendrocytes, resulting in progressive cytopathic loss. Most of MSA are sporadic cases, familial cases are seen very rarely, and genetic mutations have been identified in some of them. At present, research on the onset mechanism is being conducted using inclusion bodies and genetic factors as clues; however, it has not yet been sufficiently elucidated. Since the striatum is degenerated in MSA, anti-Parkinsonian drugs are considered to be less effective than in Parkinson's disease. In addition, since cerebellar symptoms and autonomic neuropathy are also added, MSA often shows progressive aggravation as a whole. According to the research results in Japan, it is reported that wheelchair use starts in about 5 years on average after the onset, bed rest condition occurs in about 8 years after the onset, and the duration of disease is about 9 years.

FTD is a neurodegenerative disease that develops mainly in the presenile period and causes neurodegeneration mainly in the frontal lobe and lateral lobe of the cerebrum, thus showing slow progression of personality change, behavioral disorder, aphasia, cognitive dysfunction, movement disorder, and the like. It is known that abnormal proteins such as tau, TDP-43, FUS and the like are accumulated in nerve cells of the cerebrum, but the detailed accumulation mechanism is unknown. Although it has been reported that antidepressants such as selective serotonin reuptake inhibitors and the like are effective in alleviating behavioral abnormalities, a radical therapeutic drug has not yet been established.

DLB is a degenerative dementia disease that develops mainly in the presenile or old age and exhibits parkinsonism and peculiar mental symptoms in addition to progressive cognitive dysfunction. Pathologically, it is characterized by the neuronal loss in the cerebrum and brain stem and the appearance of Lewy body, and has something in common with Parkinson's disease. It is the second most common degenerative dementia disease after AD in old age. There are reports that it is often found in men, and it mostly develops in the 50s to 70s in terms of age. Recently, there are many reports on the onset after the age of 80, rarely in the 30s and 40s. To date, there is no radical cure, and symptomatic treatments include drug therapy for cognitive dysfunction and drug therapy for core symptoms of hallucinations, parkinsonism, and the like.

VaD often develops suddenly and shows cognitive dysfunction as a result of abnormalities in brain blood vessels such as cerebral infarction, cerebral hemorrhage, and the like. It progresses as a sequelae of some kind of brain disorder, and the symptoms differ depending on the site of the disorder. Disordered functions and undisordered functions are mixed, including neurological symptoms such as paralysis, sensory impairment, and the like. In Japan, it is the second most common causative disease of dementia after AD, and the prevalence rate is about 2%. It used to be the most common dementia causative disease, but it now tends to decrease due to the advanced prevention and treatment of hypertension, diabetes, dyslipidemia, and the like. Nevertheless, it is the most common causative disease of juvenile dementia that develops under the age of 65, and accounts for about 40%.

Parkinson's disease is a progressive neurodegenerative disease that shows movement disorders such as hand tremor, difficulty in movement and walking, and the like. As it progresses, self walking becomes difficult, and the patient often becomes wheelchair-bound or bedridden in many cases. It often develops among middle-aged and older people aged 40 and over, particularly those aged 65 and over. Motor dysfunctions such as akinesia, rest tremor, muscular rigidity, and impairment of posutural reflex are the main symptoms, and the symptom often progresses slowly over time. PDD is a cognitive dysfunction associated with Parkinson's disease, and the onset rate increases with age as compared with Parkinson's disease without cognitive dysfunction. In PDD, symptoms similar to dementia such as apathy, depression, sleep disorder, delusion, and auditory hallucination appear in addition to a decline of cognitive function.

MS is a disease in which lesions are formed in places such as the brain, spinal cord, and optic nerve, and various symptoms appear. In many cases, recurrence in which symptoms occur and remission in which symptoms subside are repeated. Myelin, which covers the axial fiber of nerve cells, is disordered for some reason, and the axial fiber is exposed, that is, demyelinated, and nerve signal transduction is disordered, resulting in emergence of various symptoms. The number of patients is said to be about 2.5 million in the world, which tends to be relatively large in Europe and the United States, and relatively small in Asia and Africa. In Japan, about 13,000 patients have been reported, and the number is increasing year by year. It is also known that many MS patients develop MS in their 20s and 30s, and that the number of women is about three times higher than that of men. Common symptoms include sensory dysfunction, disorder of movement and gait, eye disorder, sexual dysfunction, emergence of mental symptoms, cognitive dysfunction due to brain atrophy, and the like. Corticosteroid hormone (steroid), which has the effect of suppressing inflammation of the lesion, is used during periods of severe symptoms, and analgesics, antiepileptic drugs, antidepressants, and the like are sometimes used as symptomatic treatments depending on the symptoms.

Tauopathy and dementia-related diseases are considered to rapidly increase after 65 years of age, and early detection and early start of the treatment are extremely important for suppressing the pathological progression by a symptomatic drug therapy. Due to the absence of a radical cure for these diseases at present, a diagnostic marker for early detection is energetically searched for. For example, in tauopathy, measurement of total tau or phosphorylated tau in cerebrospinal fluid is considered to reflect the degree of tau accumulation in the brain. However, it is difficult to measure the amount of tau in the brain or the amount of tau in the cerebrospinal fluid. In addition, it is difficult to diagnose dementia-related diseases at an early stage.

From the above, a method that can easily and highly sensitively determine patients with tauopathy and dementia-related diseases and people at risk of the disease is demanded.

S38AA, particularly an extracellular domain thereof, has been reported as a diagnostic marker for AD (Patent Literature 1). However, no disclosure or suggestion is found as to the usefulness of S38AA and an extracellular domain thereof for the diagnosis and the like of tauopathy and dementia-related diseases excluding AD.

### [Citation List]

### [Patent Literature]

[PTL 1]
WO 2012/091138

### [Summary of Invention]

### [Technical Problem]

An object of the present invention is to provide an agent for determining tauopathy and dementia-related diseases, a method for determining tauopathy and dementia-related diseases, a method for treating tauopathy and dementia-related diseases, a method for selecting a candidate substance for a therapeutic drug for tauopathy and dementia-related diseases, and the like.

### [Solution to Problem]

S38AA extracellular domain (hereinafter sometimes to be abbreviated as S38AA fragment) is known to increase in cerebrospinal fluid and plasma of Alzheimer's disease patients (hereinafter sometimes to be referred to as "AD patients"). The present inventors previously found that two kinds of S38AA fragments (S38AA short fragment (hereinafter sometimes to be abbreviated as "short fragment") and S38AA long fragment (hereinafter sometimes to be abbreviated as "long fragment") exist and that the S38AA short fragments have extremely high reliability as an index for highly accurate determination of the onset of and people at risk of Alzheimer's disease, and the degree of progression of the disease. Based on the above-mentioned findings, the present inventors have conducted further studies and found that S38AA short fragment can be used not only for Alzheimer's disease but also widely for extremely highly reliable determination of the onset of tauopathy and dementia-related diseases and people at risk of the disease, which resulted in the completion of the present invention.

### kit claims

[item 1] A kit for use in determining tauopathy or a dementia-related disease (excluding Alzheimer's disease), comprising an antibody that recognizes a polypeptide consisting of (1) the amino acid sequence shown in SEQ ID NO: 1, or (2) an amino acid sequence resulting from substitution, deletion, addition or insertion of one to several amino acids in the amino acid sequence shown in SEQ ID NO: 1.
[item 2] The kit of item 1, wherein the aforementioned polypeptide consists of the amino acid sequence shown in SEQ ID NO: 1.
[item 3] The kit of item 1 or 2, wherein the aforementioned antibody further recognizes the polypeptide of SEQ ID NO: 2.
[item 4] The kit of any one of items 1 to 3, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 5] The kit of any one of items 1 to 4, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 6] The kit of any one of items 1 to 5, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 7] The kit of any one of items 1 to 6, wherein the antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 8] The kit of any one of items 1 to 7, further comprising a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1.
[item 9] The kit of any one of items 1 to 8, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS). determination agent claims
[item 10] An agent for determining tauopathy or a dementia-related disease (excluding Alzheimer's disease), comprising an antibody capable of measuring an amount of a polypeptide consisting of (1) the amino acid sequence shown in SEQ ID NO: 1, or (2) an amino acid sequence resulting from substitution, deletion, addition or insertion of one to several amino acids in the amino acid sequence shown in SEQ ID NO: 1.
[item 11] The agent of item 10, wherein the aforementioned polypeptide consists of the amino acid sequence shown in SEQ ID NO: 1.
[item 12] The agent of item 10 or 11, wherein the aforementioned antibody further recognizes the polypeptide of SEQ ID NO: 2.
[item 13] The agent of any one of items 10 to 12, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 14] The agent of any one of items 10 to 13, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 15] The agent of any one of items 10 to 14, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 16] The agent of any one of items 10 to 15, wherein the aforementioned antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 17] The agent of any one of items 10 to 16, further comprising a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1.
[item 18] The agent of any one of items 10 to 17, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS). claims for use for the manufacture of determination agent
[item 19] Use of an antibody that recognizes a polypeptide consisting of (1) the amino acid sequence shown in SEQ ID NO: 1, or
   (2) an amino acid sequence resulting from substitution, deletion, addition or insertion of one to several amino acids in the amino acid sequence shown in SEQ ID NO: 1 in the manufacture of an agent for determining tauopathy or a dementia-related disease (excluding Alzheimer's disease).
[item 20] The use of item 19, wherein the aforementioned polypeptide consists of the amino acid sequence shown in SEQ ID NO: 1.
[item 21] The use of item 19 or 20, wherein the aforementioned antibody further recognizes the polypeptide of SEQ ID NO: 2.
[item 22] The use of any one of items 19 to 21, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 23] The use of any one of items 19 to 22, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 24] The use of any one of items 19 to 23, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 25] The use of any one of items 19 to 24, wherein the aforementioned antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 26] The kit of any one of items 19 to 25, further comprising a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1.
[item 27] The use of any one of items 19 to 26, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS). method for determining contraction possibility and the like [item 28] A method for determining whether a test animal is affected with tauopathy or a dementia-related disease at present or may be affected with tauopathy or a dementia-related disease in the future, comprising detecting a polypeptide consisting of (1) the amino acid sequence shown in SEQ ID NO: 1, or
   (2) an amino acid sequence resulting from substitution, deletion, addition or insertion of one to several amino acids in the amino acid sequence shown in SEQ ID NO: 1 in a sample collected from the test animal, wherein the aforementioned tauopathy and dementia-related disease do not include
   - Alzheimer's disease.
[item 29] The method of item 28, comprising the following steps (i) to (iii):
   (i) a step of quantifying the aforementioned polypeptide in a sample collected from a test animal,
   (ii) a step of comparing the amount of the aforementioned polypeptide quantified in (i) with the amount of the aforementioned polypeptide in a sample collected from a healthy animal (hereinafter to be referred to as control value), and
   (iii) a step of determining based on the results of (ii) that the aforementioned test animal may be affected with tauopathy or a dementia-related disease at present or that the animal may be affected with tauopathy or a dementia-related disease in the future, when the amount of the aforementioned polypeptide quantified in (i) is higher than the control value.
[item 30] The method of item 29, wherein the aforementioned amount of the aforementioned polypeptide quantified in (i) is not less than 1.1 times of the control value.
[item 31] The method of item 28, comprising the following steps (i) and (ii):
   (i) a step of quantifying the aforementioned polypeptide in a sample collected from a test animal,
   (ii) a step of determining that the aforementioned test animal may be affected with tauopathy or a dementia-related disease at present or that the animal may be affected with tauopathy or a dementia-related disease in the future when the amount of the aforementioned polypeptide quantified in (i) is higher than the cutoff value.
[item 32] The method of item 31, wherein the aforementioned cutoff value is 45 - 85 units.
[item 33] The method of item 31, wherein the aforementioned cutoff value is 45 - 85 ng/mL.
[item 34] The method of any one of items 28 to 33, wherein the aforementioned test animal is a human.
[item 35] The method of any one of items 28 to 34, wherein the aforementioned sample is blood, cerebrospinal fluid, saliva, lacrimal fluid, or urine.
[item 36] The method of any one of items 28 to 35, further comprising detecting other one or more tauopathy and dementia-related disease diagnosis markers.
[item 37] The method of any one of items 28 to 36, wherein the aforementioned polypeptide consists of the amino acid sequence shown in SEQ ID NO: 1.
[item 38] The method of any one of items 28 to 37, wherein the aforementioned polypeptide is detected using an antibody.
[item 39] The method of item 38, wherein the aforementioned antibody further recognizes the polypeptide of SEQ ID NO: 2.
[item 40] The method of any one of items 38 and 39, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 41] The method of any one of items 38 to 40, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 42] The method of any one of items 38 to 41, wherein the amino acid sequence of the heavy chain variable region of the aforementioned antibody is SEQ ID NO: 4, SEQ ID NO: 6, SEQ ID NO: 8, SEQ-ID NO: 10, SEQ ID NO: 12, SEQ ID NO: 14, SEQ ID NO: 16, SEQ ID NO: 18, SEQ ID NO: 20, or SEQ ID NO: 22, and
   the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 43] The method of any one of items 38 to 42, wherein the aforementioned antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 44] The method of any one of items 28 to 43, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).
   method for determining degree of progression
[item 45] A method for determining the degree of progression of tauopathy or a dementia-related disease (excluding Alzheimer's disease), comprising detecting a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in a sample collected from a test animal.
[item 46] The method of item 45, comprising the following steps (i) to (iii):
   (i) a step of quantifying the aforementioned polypeptide in a sample collected from a test animal that is or may be affected with tauopathy or a dementia-related disease,
   (ii) a step of comparing the amount of the aforementioned polypeptide quantified in (i) with the amount of the aforementioned polypeptide in a sample collected from an animal affected with tauopathy or the dementia-related disease at a specific degree of progression (hereinafter control value), and
   (iii) a step of determining, based on the results of (ii), that the degree of progression of the tauopathy or dementia-related disease of the aforementioned test animal is higher than that of an animal affected with the disease as a control when the amount of the aforementioned polypeptide quantified in (i) is higher than the control value, and that the degree of progression of the tauopathy or dementia-related disease of the aforementioned test animal is lower than that of an animal affected with the disease as a control when the amount is smaller than the control value.
[item 47] The method of item 45, comprising the following steps (i) to (iii):
   (i) a step of quantifying the aforementioned polypeptide in a sample collected from a test animal that is or may be affected with tauopathy or a dementia-related disease,
   (ii) a step of comparing the amount of the aforementioned polypeptide quantified in (i) with the amount of the aforementioned polypeptide in a sample collected in the past from the test animal (hereinafter control value), and
   (iii) a step of determining, based on the results of (ii), that the tauopathy or dementia-related disease of the aforementioned test animal is progressing when the amount of the aforementioned polypeptide quantified in (i) is higher than the control value, and that the tauopathy or dementia-related disease of the aforementioned test animal was improved when the amount is smaller than the control value.
[item 48] The method of any one of item 46 and item 47, wherein the tauopathy or dementia-related disease of the aforementioned test animal is determined to be progressing when the aforementioned amount of the aforementioned polypeptide quantified in (i) is not less than 1.1 times of the control value.
[item 49] The method of any one of item 46 and item 47, wherein the tauopathy or dementia-related disease of the aforementioned test animal is determined to be improved when the aforementioned amount of the aforementioned polypeptide quantified in (i) is not more than 0.9 times of the control value.
[item 50] The method of item 45, comprising the following steps (i) to (iii):
   (i) a step of quantifying the aforementioned polypeptide in-a sample collected from a test animal,
   (ii) a step of determining that tauopathy or a dementia-related disease of the test animal is exacerbated when the amount of the aforementioned polypeptide quantified in (i) is higher than the cutoff value.
[item 51] The method of any one of items 45 to 50, wherein the aforementioned test animal is a human.
[item 52] The method of any one of items 45 to 51, wherein the aforementioned sample is blood, cerebrospinal fluid, saliva, lacrimal fluid, or urine.
[item 53] The method of any one of items 45 to 52, further comprising detecting other one or more tauopathy and dementia-related disease diagnosis markers.
[item 54] The method of any one of items 45 to 53, wherein the aforementioned polypeptide consists of the amino acid sequence shown in SEQ ID NO: 1.
[item 55] The method of any one of items 45 to 54, wherein the aforementioned polypeptide is detected using an antibody.
[item 56] The method of item 55, wherein the aforementioned antibody further recognizes the polypeptide of SEQ ID NO: 2.
[item 57] The method of item 55 or 56, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 58] The method of any one of items 55 to 57, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 59] The method of any one of items 55 to 58, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 60] The method of any one of items 55 to 59, wherein the antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 61] The method of any one of items 45 to 60, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).
   method for treatment or prophylaxis
[item 62] A method for treating or preventing tauopathy or a dementia-related disease, comprising detecting a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in a sample collected from a test animal, and administering a therapeutic drug for tauopathy and dementia-related diseases to the test animal, wherein the aforementioned tauopathy and dementia-related diseases do not include Alzheimer's disease.
[item 63] The method of item 62, comprising the following steps (i) to (iv):
   (i) a step of quantifying the polypeptide of any one of items 1 and 2 in a sample collected from a test animal,
   (ii) a step of comparing the amount of the aforementioned polypeptide quantified in (i) with the amount of the aforementioned polypeptide in a sample collected from a healthy animal (hereinafter to be referred to as control value),
   (iii) a step of determining, based on the results of (ii), that the aforementioned test animal is or may be affected with tauopathy or a dementia-related disease at present, or may be affected with tauopathy or a dementia-related disease in the future when the amount of the aforementioned polypeptide quantified in (i) is higher than the control value, and
   (iv) a step of administering, based on the results of (iii), a therapeutic or prophylactic drug for tauopathy and dementia-related diseases to a test animal determined to be affected or possibly affected with tauopathy or a dementia-related disease at present, or possibly affected with tauopathy or a dementia-related disease in the future.
[item 64] The method of item 63, wherein the aforementioned amount of the aforementioned polypeptide quantified in (i) is not less than 1.1 times of the control value.
[item 65] The method of item 62, comprising the following steps (i) and (ii):
   (i) a step of quantifying the aforementioned polypeptide in a sample collected from a test animal,
   (ii) a step of determining that the aforementioned test animal may be affected with tauopathy or a dementia-related disease at present or that the animal may be affected with tauopathy or a dementia-related disease in the future when the amount of the aforementioned polypeptide quantified in (i) is higher than the cutoff value.
[item 66] The method of item 65, wherein the aforementioned cutoff value is 45 - 85 units.
[item 67] The method of item 65, wherein the aforementioned cutoff value is 45 - 85 ng/mL.
[item 68] The method of any one of items 62 to 67, wherein the aforementioned test animal is a human.
[item 69] The method of any one of items 62 to 68, wherein the aforementioned sample is blood, cerebrospinal fluid, saliva, lacrimal fluid, or urine.
[item 70] The method of any one of items 62 to 69, further comprising detecting other one or more tauopathy and dementia-related disease diagnosis markers.
[item 71] The method of any one of items 62 to 70, wherein the aforementioned polypeptide consists of the amino acid sequence shown in SEQ ID NO: 1.
[item 72] The method of any one of items 62 to 71, wherein the aforementioned polypeptide is detected using an antibody.
[item 73] The method of item 72, wherein the aforementioned antibody further recognizes the polypeptide of SEQ ID NO: 2.
[item 74] The method of any one of items 72 to 73, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 75] The method of any one of items 72 to 74, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 76] The method of any one of items 72 to 75, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 77] The method of any one of items 72 to 76, wherein the antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 78] The method of any one of items 62 to 77, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).
[item 79] The method of any one of items 62 to 78, wherein the therapeutic drug for tauopathy or a dementia-related disease is selected from the group consisting of cholinesterase inhibitor, NMDA receptor antagonist, tau protein remover and production inhibitor, therapeutic drug for Parkinson's disease, and therapeutic drug for multiple sclerosis.
[item 80] The method of item 79, wherein the aforementioned cholinesterase inhibitor is at least one selected from the group consisting of donepezil, galanthamine, rivastigmine, Huperzine A, and tacrine.
[item 81] The method of item 79, wherein the aforementioned NMDA receptor antagonist is memantine.
[item 82] The method of item 79, wherein the aforementioned tau protein remover and production inhibitor are at least one selected from the group consisting of tau protein vaccine, tau protein removing antibody, tau protein modifying inhibitor, tau protein coagulation inhibitor, and tau proteolysis promoter.
[item 83] The method of item 82, wherein the aforementioned tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.
[item 84] The method-of item 79, wherein the aforementioned therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.
[item 85] The method of item 79, wherein the aforementioned therapeutic drug for multiple sclerosis is at least one selected from the group consisting of steroid, interferon β, glatirameracetate, fingolimod, natalizumab, MN-166, siponimod, laquinimod, and masitinib. medication method claim
[item 86] A method for administering a medicine for the treatment or prophylaxis of tauopathy or a dementia-related disease, comprising quantifying a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in a sample collected from a test animal, selecting a therapeutic or prophylactic drug for tauopathy or a dementia-related disease, and administering the therapeutic drug for tauopathy or a dementia-related disease to the test animal, wherein the aforementioned tauopathy and dementia-related disease do not include Alzheimer's disease.
[item 87] The method of item 86, comprising
   (i) a step of quantifying the aforementioned polypeptide in a sample collected from a test animal that is or may be affected with tauopathy or a dementia-related disease at present,
   (ii) a step of comparing the amount of the aforementioned polypeptide quantified in (i) with the amount of the aforementioned polypeptide in a sample collected in the past from the test animal (hereinafter control value),
   (iii) a step of determining, based on the results of (ii), that the tauopathy or dementia-related disease of the aforementioned test animal is progressing when the amount of the aforementioned polypeptide quantified in (i) is higher than the control value, and that the tauopathy or dementia-related disease of the aforementioned test animal was improved when the amount is smaller than the control value,
   (iv) a step of selecting a therapeutic or prophylactic drug for tauopathy or a dementia-related disease based on the results of (iii), and
   (v) a step of administering the therapeutic drug for tauopathy or a dementia-related disease selected in (iv) to a test animal.
[item 88] The method of item 87, wherein the tauopathy or a dementia-related disease in the aforementioned test animal is determined to be progressing when the aforementioned amount of the aforementioned polypeptide quantified in (i) is not less than 1.1 times the control value.
[item 89] The method of item 87, wherein the tauopathy or a dementia-related disease in the aforementioned test animal is determined to be improving when the aforementioned amount of the aforementioned polypeptide quantified in (i) is not more than 0.9 times the control value.
[item 90] The method of any one of items 86 to 89, wherein the aforementioned test animal is a human.
[item 91] The method of any one of items 86 to 90, wherein the aforementioned sample is blood, cerebrospinal fluid, saliva, lacrimal fluid or urine.
[item 92] The method of any one of items 86 to 91, further comprising detecting other one or more tauopathy and dementia-related disease diagnosis markers.
[item 93] The method of any one of items 86 to 92, wherein the aforementioned polypeptide consists of the amino acid sequence shown in SEQ ID NO: 1.
[item 94] The method of any one of items 86 to 93, wherein the aforementioned polypeptide is detected using an antibody.
[item 95] The method of item 94, wherein the aforementioned antibody further recognizes the polypeptide of SEQ ID NO: 2.
[item 96] The method of item 94 or 95, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 97] The method of any one of items 94 to 96, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 98] The method of any one of items 94 to 97, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 99] The method of any one of items 94 to 98, wherein the antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 100] The method of any one of items 86 to 99, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).
[item 101] The method of any one of items 86 to 100, wherein the therapeutic drug for tauopathy or a dementia-related disease is selected from the group consisting of cholinesterase inhibitor, NMDA receptor antagonist, and tau protein remover and production inhibitor.
[item 102] The method of item 101, wherein the aforementioned cholinesterase inhibitor is at least one selected from the group consisting of donepezil, galanthamine, rivastigmine, Huperzine A, and tacrine.
[item 103] The method of item 101, wherein the aforementioned NMDA receptor antagonist is memantine.
[item 104] The method of item 104, wherein the aforementioned tau protein remover and production inhibitor are at least one selected from the group consisting of tau protein vaccine, tau protein removing antibody, tau protein modifying inhibitor, tau protein coagulation inhibitor, and tau proteolysis promoter.
[item 105] The method of item 101, wherein the aforementioned tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.
[item 106] The method of item 101, wherein the aforementioned therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.
[item 107] The method of item 101, wherein the aforementioned therapeutic drug for multiple sclerosis is at least one selected from the group consisting of steroid, interferon β, glatirameracetate, fingolimod, natalizumab, MN-166, siponimod, laquinimod, and masitinib. therapeutic drug claim
[item 108] A therapeutic drug for tauopathy or a dementia-related disease for use for a patient with determined degree of progression of tauopathy or the dementia-related disease after quantifying a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in a sample collected from a test animal, wherein the aforementioned tauopathy and dementia-related disease do not include Alzheimer's disease.
[item 109] The therapeutic drug of item 108 for use for a patient whose degree of progression of tauopathy and dementia-related diseases has been determined by the following steps (i) to (iv):
   (i) a step of quantifying the aforementioned polypeptide in a sample collected from a test animal that is or may be affected with tauopathy or a dementia-related disease at present,
   (ii) a step of comparing the amount of the aforementioned polypeptide quantified in (i) with the amount of the aforementioned polypeptide in a sample collected in the past from the test animal (hereinafter control value),
   (iii) a step of determining, based on the results of (ii), that the tauopathy or dementia-related disease of the aforementioned test animal is progressing when the amount of the aforementioned polypeptide quantified in (i) is higher than the control value, and that the tauopathy or dementia-related disease of the aforementioned test animal was improved when the amount is smaller than the control value, and
   (iv) a step of determining, based on the results of (iii), administration of the therapeutic drug for tauopathy or a dementia-related disease.
[item 110] The therapeutic drug of item 109, wherein the tauopathy or dementia-related disease of the aforementioned test animal is determined to be progressing when the aforementioned amount of the aforementioned polypeptide quantified in (i) is not less than 1.1 times of the control value.
[item 111] The therapeutic drug of item 109, wherein the tauopathy or dementia-related disease of the aforementioned test animal is determined to be improved when the aforementioned amount of the aforementioned polypeptide quantified in (i) is not more than 0.9 times of the control value.
[item 112] A therapeutic drug for tauopathy or a dementia-related disease for use for a patient determined to be possibly affected with tauopathy or the dementia-related disease at present or possibly affected with tauopathy or the dementia-related disease in the future:
   (i) a step of quantifying the aforementioned polypeptide in a sample collected from a test animal that is or may be affected with tauopathy or a dementia-related disease at present,
   (ii) a step of determining that the aforementioned test animal may be affected with tauopathy or a dementia-related disease at present or that the animal may be affected with tauopathy or a dementia-related disease in the future when the amount of the aforementioned polypeptide quantified in (i) is higher than the cutoff value.
   (iii) a step of determining, based on the results of (ii), administration of the therapeutic drug for tauopathy or a dementia-related disease, wherein the aforementioned tauopathy and dementia-related disease do not include Alzheimer's disease.
[item 113] The therapeutic drug of item 112, wherein the aforementioned cutoff value is 45 - 85 units.
[item 114] The therapeutic drug of item 112, wherein the aforementioned cutoff value is 45 - 85 ng/mL.
[item 115] The therapeutic drug of any one of items 108 to 114, wherein the aforementioned test animal is a human.
[item 116] The therapeutic drug of any one of items 108 to 115, wherein the aforementioned sample is blood, cerebrospinal fluid, saliva, lacrimal fluid or urine.
[item 117] The therapeutic drug of any one of items 108 to 116, further comprising detecting other one or more tauopathy and dementia-related disease diagnosis markers.
[item 118] The therapeutic drug of any one of items 108 to 117, wherein the aforementioned polypeptide consists of the amino acid sequence shown in SEQ ID NO: 1.
[item 119] The therapeutic drug of any one of items 108 to 118, wherein the aforementioned polypeptide is detected using an antibody.
[item 120] The therapeutic drug of item 119, wherein the aforementioned antibody further recognizes the polypeptide of SEQ ID NO: 2.
[item 121] The therapeutic drug of any one of items 119 and 120, wherein the aforementioned antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 122] The therapeutic drug of any one of items 119 to 121, wherein the aforementioned antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 123] The therapeutic drug of any one of items 119 to 122, wherein the amino acid sequence of the heavy chain variable region of the aforementioned antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
   the amino acid sequence of the light chain variable region of the aforementioned antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.
[item 124] The therapeutic drug of any one of items 119 to 123, wherein the antibody is
   (1) an antibody comprising the heavy chain variable region of SEQ ID NO: 4, and the light chain variable region of SEQ ID NO: 5,
   (2) an antibody comprising the heavy chain variable region of SEQ ID NO: 6, and the light chain variable region of SEQ ID NO: 7,
   (3) an antibody comprising the heavy chain variable region of SEQ ID NO: 8, and the light chain variable region of SEQ ID NO: 9,
   (4) an antibody comprising the heavy chain variable region of SEQ ID NO: 10, and the light chain variable region of SEQ ID NO: 11,
   (5) an antibody comprising the heavy chain variable region of SEQ ID NO: 12, and the light chain variable region of SEQ ID NO: 13,
   (6) an antibody comprising the heavy chain variable region of SEQ ID NO: 14, and the light chain variable region of SEQ ID NO: 15,
   (7) an antibody comprising the heavy chain variable region of SEQ ID NO: 16, and the light chain variable region of SEQ ID NO: 17,
   (8) an antibody comprising the heavy chain variable region of SEQ ID NO: 18, and the light chain variable region of SEQ ID NO: 19,
   (9) an antibody comprising the heavy chain variable region of SEQ ID NO: 20, and the light chain variable region of SEQ ID NO: 21, or
   (10) an antibody comprising the heavy chain variable region of SEQ ID NO: 22, and the light chain variable region of SEQ ID NO: 23.
[item 125] The therapeutic drug of any one of items 108 to 124, wherein the aforementioned tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).
[item 126] The therapeutic drug of any one of items 108 to 125, wherein the therapeutic drug for tauopathy or a dementia-related disease is selected from the group consisting of cholinesterase inhibitor, NMDA receptor antagonist, amyloid β remover and production inhibitor, and Tau protein remover and production inhibitor.
[item 127] The therapeutic drug of item 126, wherein the aforementioned cholinesterase inhibitor is at least one selected from the group consisting of donepezil, galanthamine, rivastigmine, Huperzine A, and tacrine.
[item 128] The therapeutic drug of item 126, wherein the aforementioned NMDA receptor antagonist is memantine.
[item 129] The therapeutic drug of item 126, wherein the aforementioned tau protein remover and production inhibitor are at least one selected from the group consisting of tau protein vaccine, tau protein removing antibody, tau protein modifying inhibitor, tau protein coagulation inhibitor, and tau proteolysis promoter.
[item 130] The therapeutic drug of item 129, wherein the aforementioned tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.
[item 131] The therapeutic drug of item 126, wherein the aforementioned therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.
[item 132] The therapeutic drug of item 126, wherein the aforementioned therapeutic drug for multiple sclerosis is at least one selected from the group consisting of steroid, interferon β, glatirameracetate, fingolimod, natalizumab, MN-166, siponimod, laquinimod, and masitinib. therapeutic or prophylactic drug - 2
[item 133] A therapeutic or prophylactic drug for tauopathy or a dementia-related disease, comprising, as an active ingredient, a medicament capable of decreasing an amount of a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in the body of a patient with tauopathy or a dementia-related disease or a person possibly affected with tauopathy or a dementia-related disease, or a medicament capable of inhibiting the production of the polypeptide in the body of a patient with tauopathy or a dementia-related disease or a person possibly affected with tauopathy or a dementia-related disease, wherein the aforementioned tauopathy and dementia-related disease do not include Alzheimer's disease.
[item 134] The drug of item 133, wherein the amount of the aforementioned polypeptide is detected using an antibody.
   method for selecting candidate substance
[item 135] A method for selecting a candidate substance for a therapeutic or prophylactic drug for tauopathy or a dementia-related disease, comprising using, as an index, reduction, by a test substance, of an amount of a polypeptide consisting of
   (1) the amino acid sequence shown in SEQ ID NO: 1, or
   (2) an amino acid sequence resulting from substitution, deletion, addition or insertion of one to several amino acids in the amino acid sequence shown in SEQ ID NO: 1,
      in the body of a patient with tauopathy or a dementia-related disease or a person possibly affected with tauopathy or a dementia-related disease, or
      inhibition, by a test substance, of the production of the polypeptide in the body of a patient with tauopathy or a dementia-related disease or a person possibly affected with tauopathy or a dementia-related disease, wherein the aforementioned tauopathy and dementia-related disease do not include Alzheimer's disease.
[item 136] The method of item 135, wherein a decrease in the amount of the aforementioned polypeptide is detected using an antibody.

### [Advantageous Effects of Invention]

According to the present invention, an agent for determining tauopathy and dementia-related diseases, a method for determining tauopathy and dementia-related diseases, a method for treating tauopathy and dementia-related diseases, a method for selecting a candidate compound for a therapeutic drug for tauopathy and dementia-related diseases, a novel anti-S38AA antibody, and the like can be provided. As used herein, tauopathy and dementia-related diseases are characterized in that they do not include Alzheimer's disease.

### [Brief Description of Drawings]

[Fig. 1]

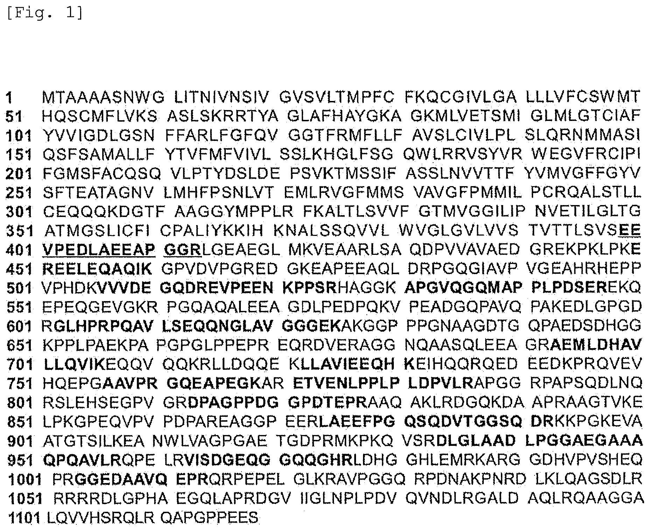

   Fig. 1 shows the amino acid sequence of S38AA. The sequence of the peptide detected by the LC-MS/MS method in identifying the N-terminal of the S38AA long fragment is shown in bold letters.
[Fig. 2]

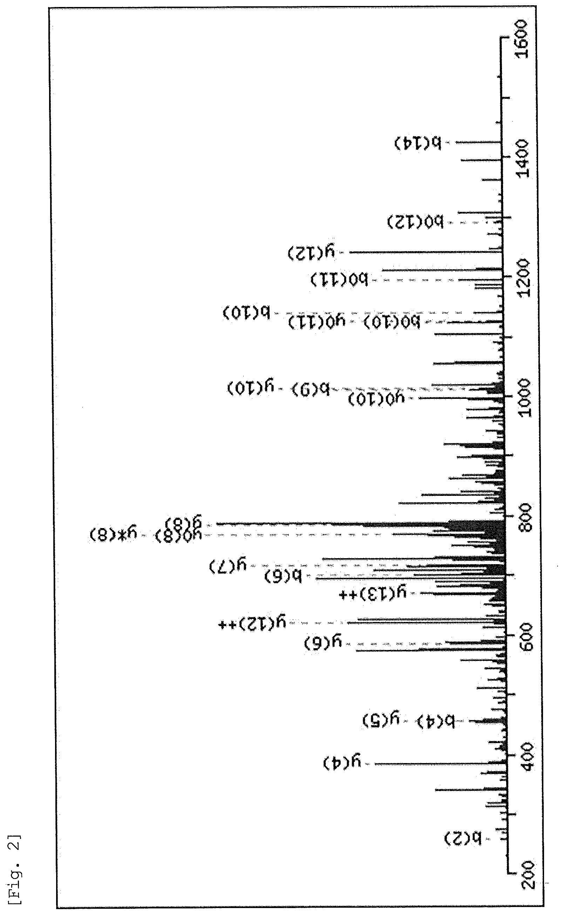

   Fig. 2 shows MS/MS peak spectrum of ³⁹⁹EEVPEDLAEEAPGGR⁴¹³ peptide detected by the LC-MS/MS method in identifying the N-terminus of the S38AA long fragment. The vertical axis shows the peak intensity of each ion, and the horizontal axis shows m/z.
[Fig. 3]

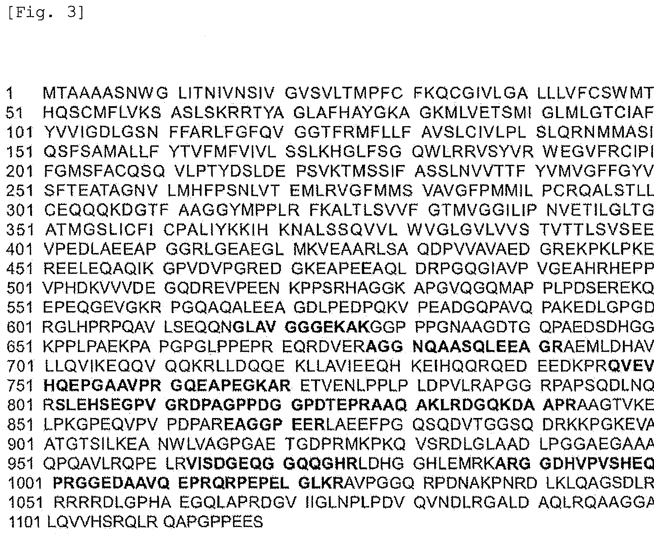

   Fig. 3 shows the amino acid sequence of S38AA. The sequence of the peptide detected by the LC-MS/MS method in identifying the N-terminus of the S38AA short fragment is shown in bold letters.
[Fig. 4]

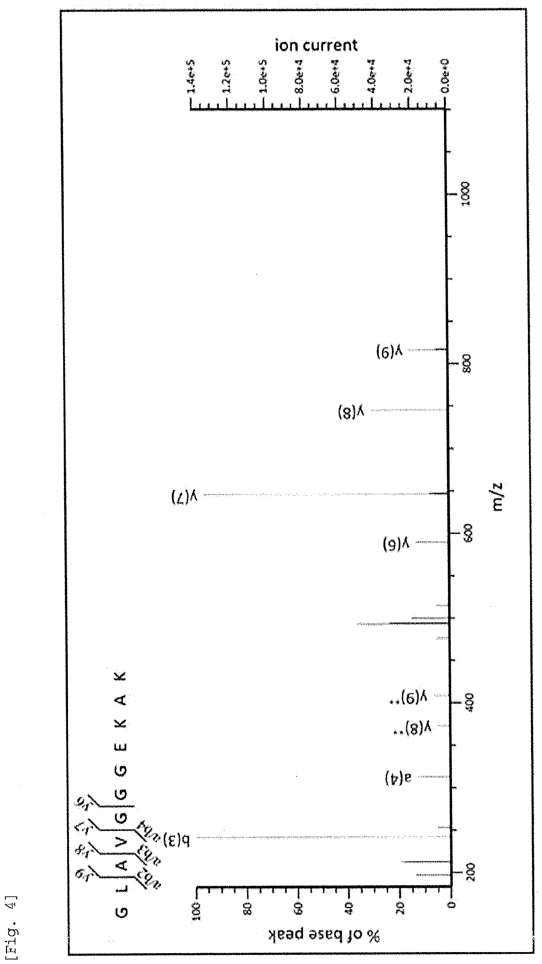

   Fig. 4 shows the MS/MS peak spectrum of ⁶¹⁷GLAVGGGEKAK⁶²⁷ peptide detected by the LC-MS/MS method in identifying the N-terminus of the S38AA short fragment. The vertical axis shows the peak intensity of each ion, and the horizontal axis shows m/z.
[Fig. 5]

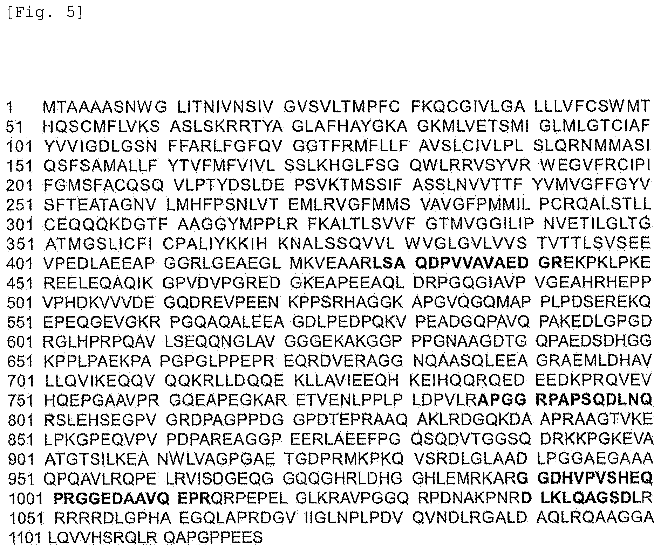

   Fig. 5 shows the amino acid sequence of S38AA. The sequence of peptide detected by the LC-MS/MS method in identifying the C-terminus of the S38AA long fragment is shown in bold letters.
[Fig. 6]

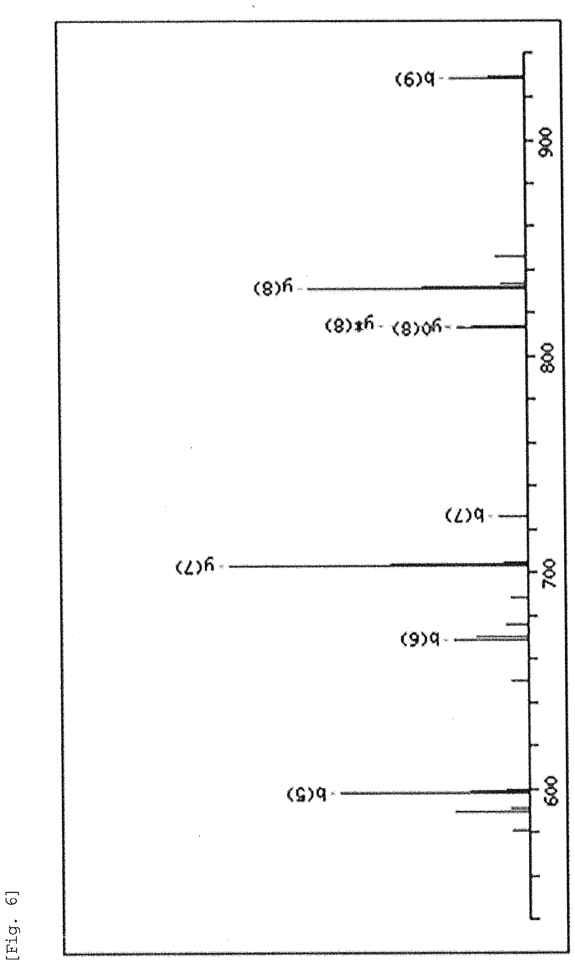

   Fig. 6 shows MS/MS peak spectrum of ¹⁰⁴⁰DLKLQAGSDL¹⁰⁴⁹ peptide detected by the LC-MS/MS method in identifying the C-terminus of the S38AA long fragment. The vertical axis shows the peak intensity of each ion, and the horizontal axis shows m/z.
[Fig. 7]

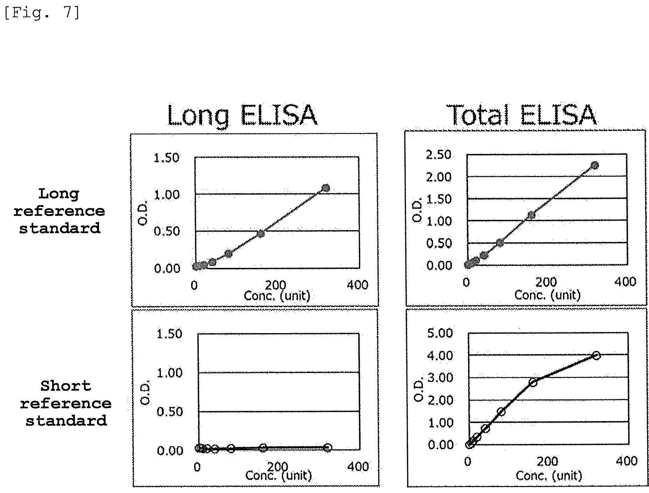

   Fig. 7 shows the quantitativeness (calibration curve) when the expression levels of recombinant proteins of S38AA long fragment and S38AA short fragment in Escherichia coli were measured by Long ELISA and Total ELISA measurement systems, respectively. The vertical axis of each graph shows the absorbance at 450 nm, and the horizontal axis shows the concentration of each protein.
[Fig. 8]

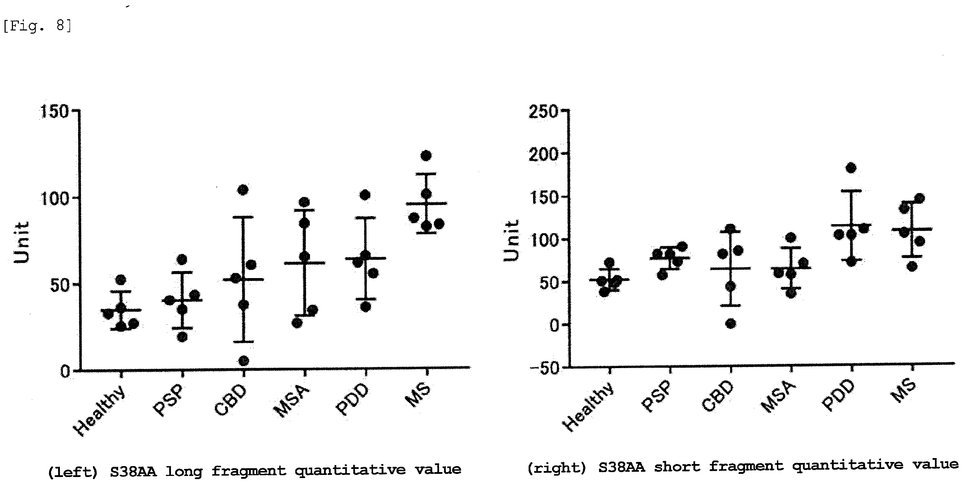

   Fig. 8 shows the quantitative values in each sample when the S38AA long fragment and the S38AA short fragment in human plasma were quantified by the subtraction method. The vertical axis shows the quantitative values of S38AA long fragment and S38AA short fragment, and the horizontal axis shows each disease name (normal, PSP, CBD, MSA, PDD, MS).
[Fig. 9]

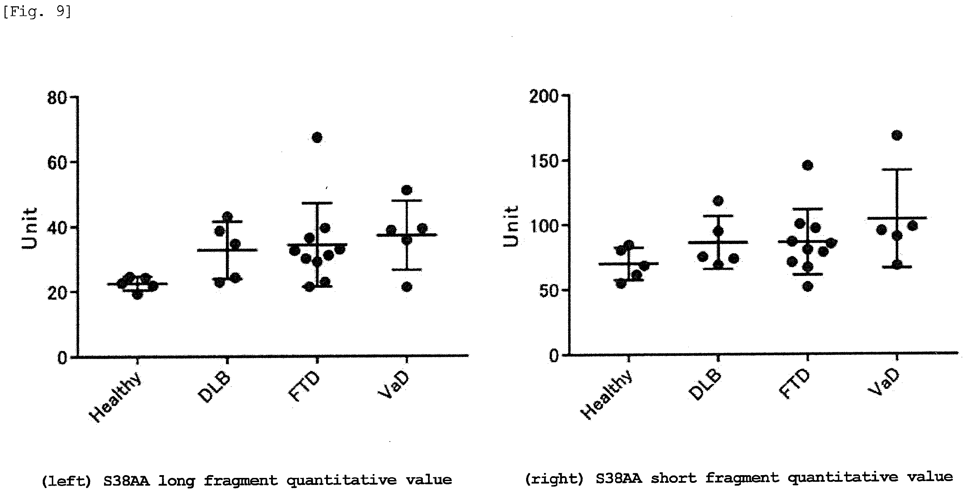

   Fig. 9 shows the quantitative values in each sample when the S38AA long fragment and the S38AA short fragment in human plasma were quantified by the subtraction method. The vertical axis shows the quantitative values of S38AA long fragment and S38AA short fragment, and the horizontal axis shows each disease name (normal, DLB, FTD, VaD).
[Fig. 10]

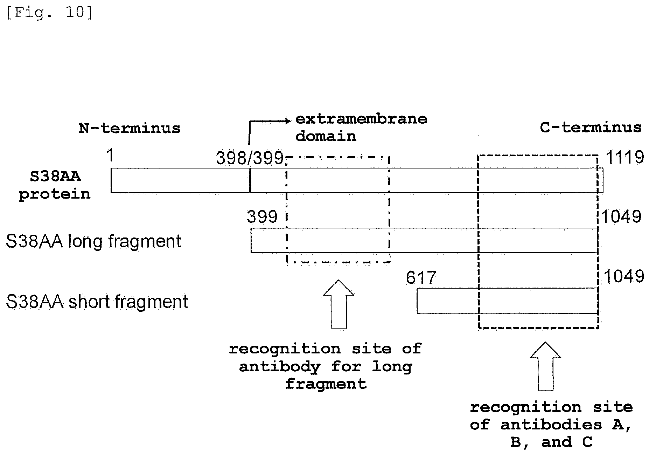

   Fig. 10 shows recognition sites of antibody for long fragment, antibody A, antibody B and antibody C.

### [Description of Embodiments]

In the present specification, "being affected with tauopathy or a dementia-related disease", that is, "being a patient with tauopathy or a dementia-related disease" refers to a condition that can be diagnosed as having developed tauopathy or a dementia-related disease by clinical diagnosis based on memory and cognitive dysfunction or image diagnosis based on brain atrophy, accumulation of tau protein, and the like.

In the present specification, "with tauopathy or a dementia-related disease" refers to a condition diagnosed as having developed tauopathy or a dementia-related disease based on each clinical diagnosis standard for tauopathy and dementia-related diseases, for example, the clinical diagnosis standard of National Institute of Neurological Disorders and Stroke and Society for PSP (NINDS-SPSP), or the like.

In all diagnostic criteria, the presence of cognitive dysfunction centering on memory disorder, slow onset and progressive process, impaired social life and activities of daily living which are associated with cognitive impairment, and differentiation/exclusion of AD type dementia, and the like are indicators of diagnosis.

The severity can be evaluated using Mini Mental State Examination (MMSE), which measures the degree of cognitive dysfunction, Functional Assessment Staging (FAST) of dementia that determines the severity mainly based on activities of daily living, Clinical Dementia Rating (CDR) that clinically determines the severity, as well as Severe Impairment Battery (SIB) and modified Rankin Scale (mRS) used in clinical test, and the like.

In the present specification, the "people at risk of tauopathy and dementia-related diseases", that is, "a person who may be affected with tauopathy and dementia-related diseases (human)", "one (human) in high risk group of tauopathy and dementia-related diseases" include humans in a state where abnormal accumulation of tau protein in the brain tissue has started, and tauopathy or a dementia-related disease is highly likely developed in the near future, that is, a state in which tau protein is accumulated in brain tissue, even though the onset of the disease cannot be diagnosed according to the aforementioned diagnosis. Here, the accumulation of tau protein in the brain tissue can be confirmed by Positron Emission Tomography (PET) of tau protein or using tau, phosphorylated tau in the cerebral spinal fluid, and the like as biomarkers.

In the present specification, the "tauopathy and dementia-related diseases" specifically refers to at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), senile dementia of the NFT type (SD-NFT), argyrophilic grain dementia (AGD), basophilic inclusion body disease (BIBD), Neuronal intermediate filament inclusion disease (NIFID), multiple sclerosis (MS), cognitive dysfunction associated with Parkinson's disease (PDD), cognitive dysfunction associated with amyotrophic lateral sclerosis (ALS), cognitive dysfunction associated with Huntington's disease (HD), and cognitive dysfunction associated with spinocerebella degeneration (SCD). In the present specification, the "tauopathy and dementia-related diseases" does not include Alzheimer's disease (AD).

As the "tauopathy and dementia-related diseases", progressive supranuclear palsy (PSP), corticobasal degeneration (CBD), multiple system atrophy (MSA), frontotemporal dementia (FTD), dementia with Lewy Bodies (DLB), vascular dementia (VaD), multiple sclerosis (MS), and cognitive dysfunction associated with Parkinson's disease (PDD) can be advantageously mentioned.

As the "tauopathy and dementia-related diseases", progressive supranuclear palsy (PSP), multiple system atrophy (MSA), frontotemporal dementia (FTD), dementia with Lewy Bodies (DLB), vascular dementia (VaD), multiple sclerosis (MS), and cognitive dysfunction associated with Parkinson's disease (PDD) can be more advantageously mentioned.

As the "tauopathy and dementia-related diseases", progressive supranuclear palsy (PSP), frontotemporal dementia (FTD), dementia with Lewy Bodies (DLB), vascular dementia (VaD), multiple sclerosis (MS), and cognitive dysfunction associated with Parkinson's disease (PDD) can be further advantageously mentioned.

As the "tauopathy and dementia-related diseases", progressive supranuclear palsy (PSP), dementia with Lewy Bodies (DLB), vascular dementia (VaD), multiple sclerosis (MS), and cognitive dysfunction associated with Parkinson's disease (PDD) can be most advantageously mentioned.

### 1. Polypeptide used in the present invention

The present inventors have found that, in the blood of patients with tauopathy and dementia-related diseases, the amount of S38AA short fragment resulting from the presence of an enzyme that specifically cleaves the C-terminal side of S38AA or an S38AA long fragment generated by cleavage in the extramembrane part of S38AA, or resulting from a high activity of the enzyme, significantly increases as compared with the amount of the S38AA long fragment, and thus found that the amount of the S38AA short fragment is extremely highly reliable as an index for the highly accurate determination of the onset of and people at risk of tauopathy and dementia-related diseases.

Therefore, the present invention provides measurement of a polypeptide consisting of the amino acid sequence shown in SEQ ID NO: 1 as the S38AA short fragment (corresponding to the 617th - 1049th amino acid sequence in the amino acid sequence (1 - 1119) of S38AA shown in SEQ ID NO: 3), and a polypeptide consisting of the amino acid sequence shown in SEQ ID NO: 2 as the S38AA long fragment (corresponding to the 399th - 1049th amino acid sequence in the amino acid sequence (1 - 1119) of S38AA shown in SEQ ID NO: 3).

As long as recognized by an antibody that specifically binds to the polypeptide consisting of the amino acid sequence shown in the below-mentioned SEQ ID NO: 1, 1 to several amino acids in the amino acid sequence of S38AA short fragment may be substituted, deleted, added or inserted. Similarly, as long as recognized by an antibody that specifically binds to the polypeptide consisting of the amino acid sequence shown in the below-mentioned SEQ ID NO: 2, 1 to several amino acids in the amino acid sequence of S38AA long fragment may be substituted, deleted, added or inserted. As used herein, several is not particularly limited and may be, for example, 2 - 10, 2 - 8, 2 - 6, 2 - 4, 2 - 3, or 2.

The S38AA short fragment may be modified at its N-terminal, C-terminal, or side chain by a method well known to those skilled in the art, for example, N-terminal acetyl group modification, C-terminal amide group modification, addition of a protein tag (His tag, etc.) to the N terminus and/or C-terminus, addition of a sugar chain to the side chain, or the like.

The polypeptide shown in the above-mentioned SEQ ID NOs: 1 to 3 (e.g., standard product, standard solution, etc.) usable in the kit, method, etc. of the present invention can be produced according to a known peptide synthesis method, for example, solid phase synthesis process, liquid phase synthesis process and the like. The obtained polypeptide can be purified and isolated by a known purification method, for example, solvent extraction, distillation, column chromatography, liquid chromatography, recrystallization, a combination of these, and the like.

The polypeptide used in the present invention mentioned above can also be produced by culturing a transformant containing the nucleic acids encoding the polypeptide and separating and purifying the polypeptide from the resulting culture. The nucleic acid used in the present invention, which encodes the polypeptide of the present invention, may be DNA, RNA, or a DNA/RNA chimera, but is preferably DNA. The nucleic acids may be double-stranded or single-stranded. In the case of double strand, it may be double-stranded DNA, double-stranded RNA or a hybrid of DNA:RNA. In the case of a single strand, it may be either a sense strand (i.e., coding strand) or an antisense strand (i.e., non-coding strand).

In the present specification, "SEQ ID NO" and "SEQ ID NO:" are synonymous. For example, "SEQ ID NO 1" and "SEQ ID NO: 1" are synonymous.

The DNA encoding the polypeptide used in the present invention mentioned above includes synthetic DNA and the like, and can be obtained by a method known per se, for example, Reverse Transcriptase-PCR method, ODA-LA PCR method, Gapped duplex method, Kunkel method and the like, or colony or plaque hybridization method or PCR method.

### 2. Anti-S38AA antibody

The "anti-S38AA antibody" used in the present specification is not particularly limited as long as it is an antibody that specifically recognizes S38AA, or an antibody that specifically recognizes S38AA long fragment and/or S38AA short fragment. For example, an antibody that recognizes the N-terminal region of S38AA long fragment, an antibody that recognizes the C-terminal region of S38AA long fragment, an antibody that recognizes the C-terminal region common to S38AA long fragment and S38AA short fragment and the like can be mentioned.

The anti-S38AA antibody may also be a commercially available anti-S38AA antibody, a polyclonal or monoclonal antibody produced by using a known method, or a fragment thereof (e.g., Fab, F(ab')₂, ScFv, minibody, etc.).

As the anti-S38AA antibody to be used in the present invention, a monoclonal antibody and a polyclonal antibody derived from mammals are preferable.

Examples of the monoclonal antibody and polyclonal antibody derived from mammals include those produced in the blood of animal, those produced by hybridomas, and those produced by a host transformed with an expression vector containing an antibody gene by a genetic engineering means, those mass-produced in CHO cells having the gene of an optimal antibody screened for from an enormous clone library consisting of 1,000,000,000,000 molecules by phage display, or human antibody directly produced using transgenic mouse that produces human antibody, and the like.

Monoclonal antibody and polyclonal antibody can be produced by a known method to those of ordinary skill in the art.

### (1) Production of monoclonal antibody

The polypeptide or a fragment thereof of the present invention is administered alone or together with a carrier or a diluent to a site where an antibody can be produced by administration to a mammal. To increase antibody producibility by administration, complete Freund's adjuvant or incomplete Freund's adjuvant may also be administered. The administration is generally performed once every 2 - 6 weeks, and about 2 - 10 times in total. Examples of the mammal to be used include monkey, rabbit, dog, guinea pig, mouse, rat, sheep and goat, with preference given to mouse and rat.

For the production of monoclonal antibody-producing cells, from mammals, for example, mice, immunized with an antigen, individuals found to show antibody titer are selected, the spleen or lymph node is collected 2 - 5 days after the final immunization, the antibody-producing cells contained therein are fused with myeloma cells, whereby a monoclonal antibody-producing hybridoma can be prepared. The antibody titer in antiserum can be measured by, for example, reacting the below-mentioned labeled S38AA with antiserum, and measuring the activity of the label bound to the antibody. A fusion operation can be performed by a known method, for example, the method of Kohler and Milstein [Nature, 256, 495 (1975)]. As the fusion stimulant, for example, polyethylene glycol (PEG), Sendai virus and the like can be mentioned, and PEG is preferably used. To enhance fusion efficiency, moreover, an adjuvant such as dimethyl sulfoxide and the like can also be used as appropriate.

As the myeloma cell, for example, NS-1, P3U1, SP2/0 and the like can be mentioned, and P3U1 is preferably used. A preferable ratio of the numbers of the antibody-producing cells (spleen cells) and myeloma cells to be used is about 1:1 - 20:1, PEG (preferably PEG 1000-PEG 6000) is added at a concentration of about 10-80%, and the cell fusion can be efficiently performed by incubating at about 20-40°C, preferably about 30 - 37°C, for about 1 - 10 min.

For screening for a monoclonal antibody-producing hybridoma, various methods can be used. Examples thereof include a method including adding a hybridoma culture supernatant to a solid phase (e.g., microplate) adsorbed with antigen such as protein and the like directly or together with carrier, adding anti-immunoglobulin antibody labeled with radioactive substance, enzyme or the like (when the cell used for cell fusion is from a mouse, anti-mouse immunoglobulin antibody is used) or protein A, and detecting monoclonal antibody bound to the solid phase, a method comprising adding a hybridoma culture supernatant to a solid phase adsorbed with anti-immunoglobulin antibody or protein A, adding protein labeled with radioactive substance, enzyme etc., and the like, and detecting monoclonal antibody bound to the solid phase, and the like.

The monoclonal antibody can be selected by a method known per se or a method analogous thereto, and can be generally selected using a medium for animal cells which is added with HAT (hypoxanthine, aminopterine, thymidine), and the like. As the medium for selection and growth, any medium can be used as long as hybridomas can grow. For example, RPMI 1640 medium containing 1 - 20%, preferably 10 - 20%, of fetal bovine serum, GIT medium containing 1 - 10% of fetal bovine serum (Wako Pure Chemical Industries, Ltd.), a serum-free medium for hybridoma culture (SFM-101, Nissui Pharmaceutical Co., Ltd.) and the like can be used. The culture temperature is generally 20 - 40°C, preferably about 37°C. The culture time is generally 5 days - 3 weeks, preferably 1 week - 2 weeks. Culture can be generally performed in 5% carbon dioxide gas. The antibody titer of the hybridoma culture supernatant can be measured in the same manner as in the above-mentioned measurement of the antibody titer of the antiserum.

The monoclonal antibody can be separated and purified according to a separation and purification method of immunoglobulin, in the same manner as in general separation and purification of polyclonal antibody [e.g., salting-out method, alcohol precipitation method, isoelectric point precipitation method, electrophoresis, adsorption and desorption method by ion exchanger (e.g., DEAE), ultracentrifugation method, gel filtration method, specific purification method including collecting only an antibody by an active adsorbent such as antigen-bound solid phase, protein A, protein G or the like, and dissociating the bond to give the antibody].

### (2) Production of polyclonal antibody

Polyclonal antibody to the polypeptide used in the present invention can be produced by a method known per se or a method analogous thereto. For example, a polyclonal antibody can be produced by producing a complex of an immunizing antigen (antigen such as protein and the like) and a carrier protein, immunizing a mammal in the same manner as in the above-mentioned production method of the monoclonal antibody or chicken, collecting a substance containing the antibodies to S38AA from the immunized animal, and separating and purifying the antibodies.

As for the complex of an immunizing antigen and a carrier protein to be used for immunizing a mammal or chicken, the kind of the carrier protein and the mixing ratio of the carrier and hapten may be any and any ratio as long as the antibody can be efficiently produced against hapten crosslinked with the carrier used for immunization. For example, a method including coupling bovine serum albumin, bovine thyroglobulin, keyhole limpet hemocyanin and the like at a weight ratio of about 0.1 - 20, preferably about 1 - 5, to hapten of 1 is used.

While various condensing agents can be used for coupling hapten with a carrier, an activated ester reagent containing glutaraldehyde, carbodiimide, maleimide activated ester, a thiol group and a dithiopyridyl group, and the like can be used.

The condensed product is administered to a mammal or chicken alone or together with a carrier and a diluent to a site where antibody can be produced. To increase antibody producibility by administration, a complete Freund's adjuvant or incomplete Freund's adjuvant may also be administered. The administration is generally performed once every 2 - 6 weeks, and about 3 - 10 times in total.

The polyclonal antibody can be collected from the blood, ascites, breast milk and the like of the mammal immunized by the above-mentioned method, preferably from the blood, and in the case of chicken, it can be collected from the blood and egg-yolk.

The titer of the polyclonal antibody in the antiserum can be measured in the same manner as in the above-mentioned measurement of the antibody titer of the antiserum. The polyclonal antibody can be separated and purified according to a separation and purification method of immunoglobulin, in the same manner as in the above-mentioned separation and purification of monoclonal antibody.

As the polyclonal antibody in the present invention, for example, rabbit-derived anti-S38AA polyclonal antibody (hereinafter to be also referred to as "MBL" or "antibody for long fragment") can be mentioned. The antibody for long fragment is characterized in that it specifically recognizes S38AA long fragment and does not recognize S38AA short fragment. The antibody for long fragment can be used for Long ELISA described later by using the antibody together with antibodies A, B, or C.

### (3) Antibody that specifically recognizes S38AA short fragment

As one embodiment of the antibody used in the present invention, an antibody that specifically recognizes the S38AA short fragment shown in SEQ ID NO: 1 can be mentioned. As an antibody that specifically recognizes the S38AA short fragment, for example, an antibody that recognizes the C-terminal sequence or the N-terminal sequence of the S38AA short fragment can be mentioned. An antibody that specifically recognizes S38AA short fragment can be prepared by a method well known to those skilled in the art. For example, it can be obtained using a peptide consisting of several amino acids from the N-terminal of the S38AA short fragment (617th glycine in the amino acid sequence shown in SEQ ID NO: 3) as an immune antigen, and selecting an antibody that is negative for a peptide which is the immune antigen added with several amino acids to its N-terminal side, or S38AA long fragment. Specifically, a monoclonal antibody or a polyclonal antibody can be obtained according to the above-mentioned method (1) or (2), respectively.

Examples of the antibody that specifically recognizes S38AA short fragment include the below-mentioned antibody A, antibody B and antibody C.

Antibody A (hereinafter to be also referred to as "mouse-derived anti-S38AA monoclonal antibody A") is an antibody that recognizes the C-terminal region of S38AA, and means an antibody obtained by establishing an antibody-producing hybridoma by using a part of the peptide of the C-terminal amino acid sequence of the S38AA fragment as an immunogen, followed by separation and purification. Antibody A is characterized in that it specifically recognizes S38AA short fragment and S38AA long fragment. Antibody A can be used for Long ELISA together with the "antibody for long fragment". Antibody A can be used for Total ELISA described later by using the antibody together with antibody B or C.

Antibody A is preferably an antibody selected from the group consisting of the following:
(1) an antibody comprising the heavy chain variable region of SEQ ID NO: 4,
(2) an antibody comprising the heavy chain variable region of SEQ ID NO: 6,
(3) an antibody comprising the heavy chain variable region of SEQ ID NO: 8, and
(4) an antibody comprising the heavy chain variable region of SEQ ID NO: 10.

Another preferred embodiment of antibody A is an antibody selected from the group consisting of the following:
(1) an antibody comprising the light chain variable region of SEQ ID NO: 5,
(2) an antibody comprising the light chain variable region of SEQ ID NO: 7,
(3) an antibody comprising the light chain variable region of SEQ ID NO: 9, and
(4) an antibody comprising the light chain variable region of SEQ ID NO: 11.

More preferably, antibody A is an antibody selected from the group consisting of the following:
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9, and
(4) an antibody comprising the heavy chain variable region of SEQ ID NO: 10, and the light chain variable region of SEQ ID NO: 11.

Antibody B is an antibody that recognizes the C-terminal region of S38AA, and means an antibody obtained by establishing an antibody-producing hybridoma by using a part of the peptide of the C-terminal amino acid sequence of the S38AA fragment as an immunogen, followed by separation and purification. Antibody B is characterized in that it specifically recognizes S38AA short fragment and S38AA long fragment. Antibody B can be used for Long ELISA together with the "antibody for long fragment". Antibody B can be used for Total ELISA by using the antibody together with antibody A or C.

Antibody B is preferably an antibody selected from the group consisting of the following:
(5) an antibody comprising the heavy chain variable region of SEQ ID NO: 12,
(6) an antibody comprising the heavy chain variable region of SEQ ID NO: 14,
(7) an antibody comprising the heavy chain variable region of SEQ ID NO: 16, and
(8) an antibody comprising the heavy chain variable region of SEQ ID NO: 18.

Another preferred embodiment of antibody B is an antibody selected from the group consisting of the following:
(5) an antibody comprising the light chain variable region of SEQ ID NO: 13,
(6) an antibody comprising the light chain variable region of SEQ ID NO: 15,
(7) an antibody comprising the light chain variable region of SEQ ID NO: 17, and
(8) an antibody comprising the light chain variable region of SEQ ID NO: 19.

Antibody B is more preferably an antibody selected from the group consisting of the following:
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17, and
(8) an antibody comprising the heavy chain variable region of SEQ ID NO: 18, and the light chain variable region of SEQ ID NO: 19.

Antibody C is an antibody that recognizes the C-terminal region of S38AA, and means an antibody obtained by establishing an antibody-producing hybridoma by using recombinant protein of the S38AA long fragment in Escherichia coli as an immunogen, followed by separation and purification. Antibody C is characterized in that it specifically recognizes S38AA short fragment and S38AA long fragment. Antibody C can be used for Long ELISA together with the "antibody for long fragment". Antibody C can be used for Total ELISA by using the antibody together with antibody A or B.

Antibody C is preferably an antibody selected from the group consisting of the following:
(9) an antibody comprising the heavy chain variable region of SEQ ID NO: 20, and
(10) an antibody comprising the heavy chain variable region of SEQ ID NO: 22.

Another preferred embodiment of antibody C is an antibody selected from the group consisting of the following:
(9) an antibody comprising the light chain variable region of SEQ ID NO: 21, and
(10) an antibody comprising the light chain variable region of SEQ ID NO: 23.

Antibody C is more preferably an antibody selected from the group consisting of the following:
(9) an antibody comprising the heavy chain variable region of SEQ ID NO: 20, and the light chain variable region of SEQ ID NO: 21, and
(10) an antibody comprising the heavy chain variable region of SEQ ID NO: 22, and the light chain variable region of SEQ ID NO: 23.

As long as the antibodies A, B and C of the present invention can specifically recognize S38AA short fragment and S38AA long fragment, the respective antibodies may contain a variable region having a homology of not less than 90%, preferably not less than 95%, more preferably not less than 96%, still more preferably not less than 97%, further preferably 98%, further more preferably not less than 99%, most preferably not less than 99.5%, with the amino acid sequence of the above-mentioned variable region contained therein.

In the present specification, "homology" means the proportion (%) of the same amino acids and similar amino acid residues to the overlapping total amino acid residues in the optimal alignment (preferably, the algorithm can consider, for the optimal alignment, introduction of a gap into one or both of the sequences), when two amino acid sequences are aligned using mathematical algorithm known in the technical field.

The homology of the amino acid sequence in the present specification can be calculated using the homology calculation algorithm NCBI BLAST (National Center for Biotechnology Information Basic Local Alignment Search Tool) under the following conditions (expectancy=10; gap; matrix =BLOSUM62; filtering=OFF). Examples of other algorithm to determine the homology of amino acid sequence include the algorithm described in Karlin et al., Proc. Natl. Acad. Sci. USA, 90:5873-5877(1993) [the algorithm is incorporated in NBLAST and XBLAST program (version 2.0) (Altschul et al., Nucleic Acids Res., 25:3389-3402(1997))], the algorithm described in Needleman et al., J. Mol. Biol., 48:444-453(1970) [the algorithm is incorporated in GAP program in GCG software package], the algorithm described in Myers and Miller, CABIOS, 4:11-17(1988) [the algorithm is incorporated in ALIGN program (version 2.0) which is a part of the CGC sequence alignment software package], the algorithm described in Pearson et al., Proc. Natl. Acad. Sci. USA, 85: 2444-2448(1988) [the algorithm is incorporated in FASTA program in the GCG software package] and the like, and they can also be preferably used in the same manner.

CDR contained in antibodies A, B and C of the present invention may contain 1 to several (2, 3, 4, 5, etc.), preferably within 2, more preferably one, amino acid substituted, deleted, inserted, and/or added in the amino acid sequence of one CDR as long as the antibodies can specifically recognize S38AA short fragment and S38AA long fragment. The number of CDRs in which amino acids of each of the above-mentioned antibodies are substituted, deleted, inserted, and/or added is not particularly limited as long as each antibody can specifically recognize S38AA short fragment and S38AA long fragment. It is preferably within 2, more preferably 1, per one light chain variable region, and preferably within 2, more preferably 1, per one heavy chain variable region. The substitution, deletion, insertion, and/or addition of the amino acids may be performed in both the light chain variable region and the heavy chain variable region, or either one alone.

For example, "similar amino acid" means amino acids similar in physicochemical properties and, for example, amino acids classified into the same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid having hydroxyl group (Ser, Thr), amino acid with small side chain (Gly, Ala, Ser, Thr, Met) and the like can be mentioned. Substitution with such similar amino acids is predicted to cause no change in the phenotype of the protein (i.e., conservative amino acid substitution). Specific examples of the conservative amino acid substitution are well known in the art and described in various documents (see, for example, Bowie et al., Science, 247:1306-1310(1990)).

Examples of the method for substituting one or more amino acid residues with other desired amino acids include Site-Directed Mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Nat1 Acad Sci USA. 82,488-492). Using the method, desired amino acids of an antibody can be substituted with other desired amino acids. It is also possible to substitute amino acids in framework and CDR with other appropriate amino acids by using library techniques such as framework shuffling (Mol Immunol. 2007 Apr; 44(11):3049-60) and CDR repair (US2006/0122377) and the like.

### 3. Agent for determining tauopathy and dementia-related diseases

The determining agent of the present invention contains one or plural kinds of anti-S38AA antibodies capable of detecting the amount of S38AA Short fragment, and can determine not only whether a person is affected with tauopathy or a dementia-related disease and whether highly likely affected with the disease at present, but also people at risk of tauopathy or a dementia-related disease, that is, whether the person has a high possibility of being affected with the disease in the near future, though the person is not yet suffering from the disease. In other words, the agent of the present invention can identify tauopathy or a dementia-related disease irrespective of the stage thereof. Therefore, the "determination" of tauopathy or a dementia-related disease in the present invention is used to mean not only determination of whether a person is already affected with tauopathy or a dementia-related disease and whether highly likely affected with the disease at present, but encompass judgment of whether a person has a high possibility of being affected in the near future, though the person is not yet suffering from the disease.

### 4. Kit for determining tauopathy and dementia-related diseases

The present invention provides a kit for determining tauopathy and dementia-related diseases. The kit of the present invention contains a reagent for measuring the amount of the S38AA short fragment. By measuring the amount of the S38AA short fragment using the kit of the present invention, tauopathy and dementia-related diseases can be determined.

The kit of the present invention contains an anti-S38AA antibody that is a single antibody or a combination of plural antibodies capable of quantifying S38AA short fragments and, specifically, an antibody that specifically recognizes S38AA short fragment, or an antibody that recognizes S38AA long fragment and does not recognize S38AA short fragment, and a combination of an antibody that recognizes both S38AA long fragment and S38AA short fragment can be mentioned. As the antibody that recognizes S38AA long fragment and does not recognize S38AA short fragment, for example, an antibody that recognizes the N-terminal side region of the aforementioned S38AA long fragment can be mentioned. As the antibody that recognizes both S38AA long fragment and S38AA short fragment, an antibody that recognizes the C-terminal side region common to S38AA long fragment and S38A short fragment can be mentioned.

The antibody used for the kit of the present invention is preferably an antibody for long fragment, antibody A, antibody B, and/or antibody C.

The antibody may be a fluorescence-labeled antibody, enzyme-labeled antibody, streptavidin-labeled antibody, biotin-labeled antibody or radioactive-labeled antibody.

The anti-S38AA antibody is generally contained in the kit of the present invention in the form of an aqueous solution thereof dissolved in water or a suitable buffer (e.g., TE buffer, PBS etc.) at a suitable concentration or a freeze-dried product.

For measurement of the S38AA short fragment and/or S38AA long fragment, one kind of antibody may be used for the measurement, and plural kinds, preferably two kinds, of antibodies may be used for the measurement (e.g., sandwich ELISA method and the like).

The kit of the present invention may further contain, in its constitution, other components necessary for performing the method, according to the measurement method of short S38AA fragment. For example, for measurement by Western blot, the kit of the present invention can further contain a blotting buffer, a labeling reagent, a blotting membrane and the like, a determination reagent, a standard solution and the like. Examples of the "standard solution" here include a purified reference standard of S38AA short fragment and/or S38AA long fragment, for example, an aqueous solution obtained by dissolving the peptide of the present invention in water or a suitable buffer (e.g., TE buffer, bovine fetal serum-containing PBS and the like) at a particular concentration.

For measurement by sandwich ELISA, the kit of the present invention can further contain, in addition to the above, an immobilized antibody measurement plate, a washing solution and the like. For measurement by an agglutination method including latex agglutination method, an antibody-coated latex, gelatin or the like can be contained. For measurement by a chemical fluorescence method or a chemical fluorescence electron method, antibody-conjugated magnetic particles and a suitable buffer can be contained. For detection of S38AA by using LC/MS, LC-MS/MS or an immunochromatography method, an antibody-coated column or micro column, and a micro chip can be contained as a part of the detection instrument. Furthermore, in a time-resolved fluorescence measurement method or a fluorescence measurement method similar thereto, a plurality of labeled anti-S38AA antibodies and other necessary components may be contained in the constitution.

As the kit of the present invention, for example, the following can be mentioned. The antibody contained in the following kits may be labeled (e.g., labeling with peroxidase, biotin, etc.). When the antibody is not labeled, a labeled antibody that labels the antibody by binding to the antibody may be contained separately.
1) one or two kinds of "antibody that specifically recognizes S38AA short fragment", washing solution, color development reagent, reaction quenching liquid, dilution buffer and standard solution;
2) one or two kinds of "antibody that recognizes the N-terminal side region of S38AA long fragment", one or two kinds of "antibody that recognizes C-terminal side region of S38AA long fragment (that is, antibody that recognizes the C-terminal side region common to S38AA long fragment and S38A short fragment)", washing solution, color development reagent, reaction quenching liquid, dilution buffer and standard solution;
3) "the first antibody that specifically recognizes S38AA short fragment", "the second antibody that specifically recognizes S38AA short fragment", washing solution, color development reagent, reaction quenching liquid, dilution buffer and standard solution;
4) "the first antibody that recognizes the N-terminal region of S38AA long fragment" and "the second antibody that specifically recognizes S38AA long fragment", one or two kinds of "antibody that recognizes C-terminal side region of S38AA long fragment (that is, antibody that recognizes the C-terminal side region common to S38AA long fragment and S38A short fragment)", washing solution, color development reagent, reaction quenching liquid, dilution buffer and standard solution.

As used herein, the "antibody that specifically recognizes S38AA short fragment", "the first antibody that specifically recognizes S38AA short fragment", and "the second antibody that specifically recognizes S38AA short fragment" are preferably antibody A, antibody B and antibody C.

As used herein, the "antibody that recognizes the N-terminal region of S38AA long fragment" and "the first antibody that recognizes N-terminal side region of S38AA long fragment" are preferably "antibody for long fragment".

As used herein, "antibody that recognizes C-terminal side region of S38AA long fragment (that is, antibody that recognizes the C-terminal side region common to S38AA long fragment and S38A short fragment)" preferably include antibody A, antibody B and antibody C. Examples of the anti-S38AA long fragment antibody and anti-S38AA short fragment antibody that can specifically detect S38AA long fragment and S38AA short fragment include the antibodies described in detail in "2. Antibody of the present invention".

### 5. Method of the present invention

### (1) Determining and test method of tauopathy and dementia-related diseases

As mentioned above, the present inventors have first found that the amount of S38AA long fragment produced by cleavage in the extramembrane part of S38AA increases in the blood of AD patients, further that, in the blood of AD patients, an enzyme that specifically cleaves the long fragment is present, or the activity of the enzyme is high, and the amount of S38AA short fragment produced by direct cleavage in the extramembrane part of S38AA increases significantly, and that the amount of S38AA short fragment in blood increases in tauopathy and dementia-related diseases, and found that the amount of the S38AA short fragment has extremely high reliability as an index for highly accurate determination of the onset of and people at risk of tauopathy or a dementia-related disease. As described above, the present inventors heretofore found that S38AA short fragments have extremely high reliability not only as an index for highly accurate determination of the onset of and people at risk of Alzheimer's disease, but also an index for determination of the degree of progression of the disease. In consideration of this point, it is sufficiently suggested that the amount of the S38AA short fragment is correlated with the degree (e.g., severe, moderate, mild, etc.) of progression of tauopathy and dementia-related diseases. As such, it is also sufficiently suggested that the amount of the S38AA short fragment in blood has extremely high reliability as an index for determination of the degree of progression of tauopathy and dementia-related diseases.

Therefore, the present invention also provides a method for determining tauopathy and dementia-related diseases, for example, progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), and cognitive dysfunction associated with Parkinson's disease (PDD), by detecting an S38AA short fragment in a sample collected from a test animal.

The determination method of the present invention can determine not only whether a person is affected with tauopathy or a dementia-related disease and whether highly likely affected with the disease at present, but also whether the person has a high possibility of being affected with the disease in the near future, even though the person is not yet suffering from the disease.

The present invention also provides a method for testing the possibility of being affected with tauopathy or a dementia-related disease at present or the possibility of being affected with tauopathy or a dementia-related disease the future, the method comprising detecting an S38AA short fragment in a sample collected from a test animal.

The above-mentioned determination and test (results) of the possibility are useful for assisting at least definitive diagnosis of tauopathy or a dementia-related disease by a doctor.

The determining and test method of the present invention is characterized by detection of S38AA short fragment in a sample collected from a test animal. In addition, the determining and test method of the present invention may include as a specific step, for example, (i) a step of quantifying S38AA short fragment in a sample collected from a test animal, and (ii) a step of comparing the amount of S38AA short fragment quantified in (i) with the amount of S38AA short fragment in a sample collected from a healthy animal (hereinafter to be referred to as "control value").

As the result of the comparison in (ii), it is shown that the aforementioned test animal is affected with tauopathy or a dementia-related disease, or may be affected with tauopathy or a dementia-related disease at present or may be affected with tauopathy or a dementia-related disease in the future, when the amount of the S38AA short fragment quantified in (i) is higher than the control value.

Furthermore, the determining and test method of the present invention may include, in addition to the above-mentioned step, a step of determining based on the results of (ii) that the aforementioned test animal is affected with tauopathy or a dementia-related disease, or may be affected with tauopathy or a dementia-related disease at present or may be affected with tauopathy or a dementia-related disease in the future, when the amount of the S38AA short fragment quantified in (i) is higher than the control value.

Furthermore, the present invention further provides a method for aiding determination of the degree of progression of tauopathy and dementia-related diseases.

The method for assisting in determining of the present invention characteristically detects S38AA short fragment in a sample collected from a test animal. The method for assisting in determining of the present invention may include as specific steps, for example, (i) a step of quantifying S38AA short fragment in a sample collected from a test animal that is or may be affected with tauopathy or a dementia-related disease, and (ii) a step of comparing the amount of the S38AA short fragment quantified in (i) with the amount of the S38AA short fragment in a sample collected in the past from the test animal (hereinafter to be referred to as "control value").

As the result of the comparison in (ii), when the amount of the S38AA short fragment quantified in (i) is higher than the control value, it indicates that the tauopathy or a dementia-related disease in the aforementioned test animal is progressing or the possibility of being affected with tauopathy or a dementia-related disease is high, and when the amount is smaller than the control value, it indicates that the tauopathy or a dementia-related disease of the aforementioned test animal is improving or the possibility of not being affected with tauopathy or a dementia-related disease is high.

The method for assisting in determining of the present invention may include, in addition to the above-mentioned steps, (iii) a step of determining based on the results of (ii) that the Alzheimer's disease in the aforementioned test animal is progressing when the amount of the S38AA short fragment quantified in (i) is higher than the control value, and the Alzheimer's disease of the aforementioned test animal is improving when the amount is smaller than the control value.

Moreover, since the amount of S38AA short fragment in blood increases in tauopathy and dementia-related diseases as described above, the present invention also provides a method for evaluating the treatment effect on patients under treatments of tauopathy and dementia-related diseases or people at risk of tauopathy and dementia-related diseases who are under treatments to prevent the onset of tauopathy and dementia-related diseases.

The method for evaluating the treatment effect of the present invention is also characterized by detection of S38AA short fragment in a sample collected from a test animal. In addition, the method for evaluating the treatment effect of the present invention may include as a specific step, for example, (i) a step of quantifying S38AA short fragment in a sample collected from a test animal that has started medication treatment of tauopathy or a dementia-related disease or medication treatment to prevent the onset of tauopathy or a dementia-related disease, and (ii) a step of comparing the amount of S38AA short fragment quantified in (i) with the amount of S38AA short fragment in a sample collected from the test animal (sample collected before the start of medication treatment, sample collected after the start of medication treatment and before the time of collection in (i)) hereinafter to be referred to as "control value").

As the result of the comparison in (ii), it is shown that the current medication treatment (or selected therapeutic drug) is not effective when the amount of the S38AA short fragment quantified in (i) higher than the control value, and that the current medication treatment (or selected therapeutic drug) is effective when the amount is smaller than the control value.

The method for evaluating the treatment effect of the present invention may include, in addition to the above-mentioned steps, (iii) a step of determining based on the results of (ii) that the current medication treatment (or selected therapeutic drug) is not effective when the amount of the S38AA short fragment quantified in (i) is higher than the control value, and the current medication treatment (or selected therapeutic drug) is effective when it is smaller than the control value.

In the present specification, the therapeutic drug used for the medication treatment is a concept that includes not only therapeutic drugs that have already been approved and marketed, but also clinical trial drugs under clinical tests. The method for evaluating the treatment effect of the present invention can also be utilized for monitoring the drug efficacy in clinical tests, and the like.

While the animal that can be a test subject for the method of the present invention is not particularly limited as long as it expresses S38AA, for example, mammals (e.g., human, monkey, bovine, swine, horse, dog, cat, sheep, goat, rabbit, hamster, guinea pig, mouse, rat etc.), birds (e.g., chicken etc.) and the like can be mentioned. Preferred is a mammal, and more preferred is a human.

While a biological sample derived from a test animal to be the sample is not particularly limited, for example, blood, serum, plasma, saliva, lacrimal fluid, urine, cerebrospinal fluid and the like can be mentioned. More preferred is plasma or cerebrospinal fluid.

Serum and plasma can be prepared by collecting blood from a test animal according to a conventional method, and separating the liquid component. The cerebrospinal fluid can be collected by a known means such as spinal tap and the like.

An S38AA long fragment and S38AA short fragment in a sample can be detected (quantified) by a known method. They can be detected by subjecting to, for example, Western blot, gel electrophoresis (e.g., SDS-PAGE, two-dimensional gel electrophoresis and the like), various separation and purification methods (e.g., ion exchange chromatography, hydrophobic chromatography, gel filtration chromatography, affinity chromatography, reversed-phase chromatography, isoelectric point chromatography, capillary electrophoresis and the like), ionization method (e.g., electron impact ionization method, field desorption method, secondary ionization method, fast atom bombardment, matrix assisted laser desorption/ionization (MALDI) method, electrospray ionization method and the like), mass spectrometer (e.g., double-focusing mass spectrometer, quadrupol mass spectrometer, time-of-flight mass spectrometer, Fourier-transform mass spectrometer, ion cyclotron mass spectrometer and the like) and the like. Detection (quantification) using a measurement device applying these measurement principles is also included in the method of the present invention.

In addition, an S38AA long fragment and S38AA short fragment can also be detected (quantified) by a known immunochemical method (nephelometry, competitive method, immunometric method, chemical fluorescence method, chemical fluorescence electron method, sandwich method etc.). As for these immunochemical methods, reference can be made to, for example, "Radioimmunoassay" edited by Hiroshi Irie (Kodansha, published in 1974), "radioimmunoassay (sequel)" edited by Hiroshi Irie (Kodansha, published in 1979), "Enzyme Immunoassay" edited by Eiji Ishikawa et al. (the 3rd edition, Igaku-Shoin, published in 1987), "Methods in ENZYMOLOGY" Vol. 121 (Immunochemical Techniques (Part I: Hybridoma Technology and Monoclonal Antibodies)) (published by Academic Press) and the like.

As a specific detection (quantification) method of S38AA. long fragment and S38AA short fragment, the amount of S38AA short fragment may be quantified by subtracting the measurement value of S38AA long fragment sample obtained using an "antibody that recognizes N-terminal side region of S38AA long fragment" alone, or an "antibody that recognizes N-terminal side region of S38AA long fragment" and an "antibody that recognizes C-terminal side region of S38AA long fragment" in combination from the measurement values of S38AA long fragment and S38AA short fragment in a sample, which are obtained using an "antibody that recognizes the C-terminal side region common to S38AA long fragment and S38A short fragment", or a measurement value obtained by a similar method may be quantified as a ratio of the amount of S38AA short fragment to the amount of S38AA long fragment, or the amount of S38AA short fragment may be directly quantified using the "antibody that specifically recognizes S38AA short fragment" of the present invention. Examples of the anti-S38AA long fragment antibody and anti-S38AA short fragment antibody capable of specifically detecting S38AA long fragment and S38AA short fragment include the antibodies described in detail in "2. Antibody of the present invention".

As used herein, the "antibody that recognizes the C-terminal side region common to S38AA long fragment and S38A short fragment" is preferably antibody A, B, or C.

As the "antibody that recognizes the N-terminal side region of S38AA long fragment" is preferably "an antibody for long fragment".

The "antibody that recognizes the C-terminal side region of S38AA long fragment" is preferably antibody A, B, or C.

The "antibody that specifically recognizes S38AA short fragment" is preferably antibody A, B, or C.

As the measurement method in the present invention, for example, "Long ELISA" can be mentioned. The "Long ELISA" is- a method for detecting (quantifying) the amount of S38AA long fragment, and is characteristically sandwich ELISA method using an "antibody for long fragment", and "at least one antibody selected from the group consisting of antibodies A, B and C".

As the measurement method in the present invention, for example, "Total ELISA" can be mentioned. The "Total ELISA" is a method for detecting (quantifying) "the total amount of S38AA long fragment and S38AA short fragment", and is characteristically sandwich ELISA method using "at least two antibodies selected from the group consisting of antibodies A, B and C".

As a "method for detecting (quantifying) the amount of S38AA short fragment" in the present invention, a method including subtracting "measurement results (quantified values) of Long ELISA" from the above-mentioned "measurement results (quantified values) of Total ELISA" can be mentioned.

As the "control value" used in the method of the present invention, the amount of S38AA short fragment in the control sample, or the amount of S38AA short fragment measured or set in advance for the control or the like may be used. It is not necessary to measure the value simultaneously with the method of the present invention.

To set the control value here, it is also possible to use a plurality of individuals as a control group and mean of the measurement values of the plurality of individuals as the control value. That is, the above-mentioned determination and the like performed using, as the control value, the amount of S38AA short fragment in the control sample derived from the control group (healthy animal, animal affected with tauopathy or a dementia-related disease in specific degree of progression, etc.) is also encompassed in the scope of the method of the present invention.-

For example, when a sample derived from a healthy animal is used as a control sample, it can be judged or determined that a person is affected with tauopathy or a dementia-related disease or has a high possibility of being affected with tauopathy or a dementia-related disease at present, or has the possibility of being affected with tauopathy or a dementia-related disease in the future.

Also, when a sample derived from an animal affected with tauopathy or a dementia-related disease in a specific degree of progression is used as a control sample, it can be judged or determined that the degree of progression of tauopathy or a dementia-related disease is higher than that of a control animal affected with the disease when the amount of S38AA short fragment in the test sample is higher than the amount in the control sample.

Furthermore, when a sample collected in the past from a test animal from which test samples were collected is used as the control sample, it can be determined that tauopathy or a dementia-related disease is progressing when the amount of S38AA short fragment in the test sample is higher than the amount in the control sample, and that the disease is improving when the amount is lower than the amount in the control sample.

Moreover, using a plurality of control samples such as a control sample derived from an animal with tauopathy or a dementia-related disease, a control sample derived from a healthy animal, and the like, it is possible to determine people at risk of tauopathy or a dementia-related disease, that is, people in whom accumulation of tau protein has started and tauopathy or a dementia-related disease is highly likely developed in the near future, even though the onset of the disease cannot be diagnosed definitely, by using the index that the amount of S38AA short fragment in the test sample is larger than that of the control sample derived from a healthy animal and smaller than that of the control sample derived from an animal with tauopathy or a dementia-related disease.

In addition, using, as a control sample, a sample collected in the past from a test animal from which test samples were collected and for which a medication treatment of tauopathy or a dementia-related disease has started, it can be evaluated that the current medication treatment is not effective when the amount of S38AA short fragment in the test sample is higher than the amount in the control sample, and that the medication treatment is effective when the amount is lower than the amount in the control sample.

When "the polypeptide of SEQ ID NO: 1 in a sample collected from a test animal" is larger than "the amount of polypeptide (control value) of SEQ ID NO: 1 in a sample collected from a healthy animal", the possibility that a test animal is affected with tauopathy or a dementia-related disease at present or the animal may be affected with tauopathy or a dementia-related disease in the future can be determined.

The amount of the "polypeptide of SEQ ID NO: 1 in a sample collected from a test animal" is preferably 1.1 to 10 times the control value. It is more preferably 1.1 to 8 times the control value. It is further preferably 1.2 to 5 times the control value. It is most preferably 1.2 to 3 times the control value.

When "the polypeptide of SEQ ID NO: 1 in a sample collected from a test animal" is larger than "the amount of aforementioned polypeptide (control value) in a sample collected in the past from a test animal", it can be determined that tauopathy or a dementia-related disease in the test animal is progressing.

The amount of the "polypeptide of SEQ ID NO: 1 in a sample collected from a test animal" is preferably 1.1 to 10 times the control value. It is more preferably 1.1 to 8 times the control value. It is further preferably 1.2 to 5 times the control value. It is most preferably 1.2 to 3 times the control value.

When "the polypeptide of SEQ ID NO: 1 in a sample collected from a test animal" is smaller than "the amount of aforementioned polypeptide (control value) in a sample collected in the past from a test animal", it can be determined that tauopathy or a dementia-related disease in the test animal is improving.

The amount of the "polypeptide of SEQ ID NO: 1 in a sample collected from a test animal" is preferably 0.1 to 0.9 times the control value. It is more preferably 0.2 to 0.9 times the control value. It is further preferably 0.3 to 0.9 times the control value. It is most preferably 0.4 to 0.9 times the control value.

Quantitative analysis of S38AA short fragment may be performed by standardizing the amount of S38AA short fragment in the sample by the amount of standard protein (internal standard protein). That is, after quantifying the amount of S38AA short fragment and the amount of standard protein in the sample by using the above-mentioned method, the ratio of the signals of the both (S38AA short fragment/standard protein) is calculated, and the amount of S38AA short fragment in the sample may be expressed as a ratio to the abundance of the standard protein.

The standard protein may be a protein that is constitutively expressed in a given amount, and a protein that is commonly expressed in many tissues and cells is preferable. For example, proteins essential for cell survival, such as proteins encoded by genes such as RNA synthase, energy-generating enzyme, ribosome protein, cellular skeleton protein and the like (housekeeping genes) can be-mentioned. Specific examples thereof include, but are not limited to, proteins such as β-actin, glyceraldehyde-3-phosphate dehydrogenase (GAPDH), β-tubulin and the like. Particularly preferred is β-actin.

In addition, instead of the aforementioned control value, a cutoff value of the amount of S38AA short fragment in blood, which relates to tauopathy and dementia-related diseases, may be set in advance, and the amount of S38AA short fragment in the blood of the test animal may be compared with the cutoff value.

For example, when the amount of the S38AA short fragment in the blood of a test animal is not less than the cutoff value, it can be determined that the test animal is affected with tauopathy or a dementia-related disease, or may be affected with tauopathy or a dementia-related disease at present or may be affected with tauopathy or a dementia-related disease in the future.

The "cutoff value" is a value that can satisfy both high diagnostic sensitivity (true positive rate) and high diagnostic specificity (true negative rate) when a disease is determined using the value as the standard. For example, a value showing a high positive rate in an individual who has developed tauopathy or a dementia-related disease and a high negative rate in an individual who has not developed tauopathy or a dementia-related disease can be set as a cutoff value.

The method for calculating the cutoff value is well known in the art. For example, the amounts of S38AA short fragment in blood in an individual who developed tauopathy or a dementia-related disease and an individual who has not developed tauopathy or a dementia-related disease are calculated, and the diagnostic sensitivity and diagnosis specificity of the calculated values are obtained. Based on these values, a ROC (Receiver Operating Characteristic) curve is created using commercially available analysis software. Then, a value at which the diagnostic sensitivity and the diagnosis specificity are as close to 100% as possible is obtained, and the value can be used as the cutoff value. In addition, for example, it is preferable to set the "mean + 2 standard deviation" of the amount of S38AA short fragment in blood of a large number of healthy animals as the cutoff value. Using this value, it can be determined with good sensitivity and specificity that tauopathy or a dementia-related disease is developed. Furthermore, for example, it is possible to determine tauopathy or a dementia-related disease with high sensitivity by obtaining a value that maximizes the likelihood ratio between the diagnostic sensitivity and the diagnosis specificity from the ROC curve and using the value as the cutoff value. Alternatively, a point with the lowest diagnosis ability on the ROC curve, that is, a point most distant from the line where the area under the ROC curve is 0.5, is set as the cutoff value, that is, "sensitivity + specificity -1" is calculated, and a point at which the value becomes the maximum value is preferably set as the cutoff value.

A cutoff value of the amount of S38AA short fragment converted with the recombinant protein of the present specification is, for example, 45 - 85 units. The cutoff value is preferably, for example, 49 - 79 units. The cutoff value is more preferably, for example, 54 - 74 units. The cutoff value is more preferably, for example, 56 - 72 units. The cutoff value is further preferably, for example, 58 - 71 units. The cutoff value is most preferably, for example, 60 - 69 units.

Another preferable value of the cutoff value includes 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, and 85 units.

A cutoff value of the amount of S38AA short fragment contained in the body is, for example, 45 - 85 ng/mL. The cutoff value is preferably, for example, 49 - 79 ng/mL. The cutoff value is more preferably, for example, 54 - 74 ng/mL. The cutoff value is more preferably, for example, 56 - 72 ng/mL. The cutoff value is further preferably, for example, 58 - 71 ng/mL. The cutoff value is most preferably, for example, 60 - 69 ng/mL.

Another preferable value of the cutoff value includes 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, and 85 ng/mL.

When determining tauopathy and dementia-related diseases by the method of the present invention, changes in other diagnostic markers for tauopathy and dementia-related diseases may be examined in addition to the S38AA short fragment. Other diagnostic markers for tauopathy and dementia-related diseases include, for example, known markers such as homocysteine, neurofilament, various inflammation-related proteins (C-reactive protein, IL-1β, TNF, IL-6 and TGFβ), cholesterol, tau, and phosphorylated tau whose potential as plasma biomarkers have been studied, and the like. These can be detected according to a conventional well-known detection method.

### (2) Method for treating or preventing tauopathy and dementia-related diseases

In the above-mentioned method of the present invention for determining tauopathy and dementia-related diseases, and the like, when the test animal is determined to be affected with tauopathy or a dementia-related disease, or possibly affected with tauopathy or a dementia-related disease at present, or possibly affected with tauopathy or a dementia-related disease in the future, tauopathy and dementia-related diseases can be treated or prevented by determining, based on the determination results, a therapeutic or prophylactic drug for tauopathy and dementia related diseases to be administered to the test animal, and administering a therapeutically effective amount of the therapeutic or prophylactic drug to the test animal.

In the present specification, the "therapeutic drug for tauopathy and dementia-related diseases" includes not only a medicament aiming at permanent cure treatment of tauopathy and dementia-related diseases but also, for example, a medicament aiming at suppressing the progression of tauopathy and dementia-related diseases. The medicament aiming at suppressing the progression may also be used as a "prophylactic drug for tauopathy and dementia-related diseases".

Examples of the therapeutic drug for tauopathy and dementia-related diseases include cholinesterase inhibitor, NMDA receptor antagonist, tau protein remover and production inhibitor, therapeutic drug for Parkinson's disease, and therapeutic drug for multiple sclerosis.

Examples of the cholinesterase inhibitor include donepezil, galanthamine, rivastigmine, huperzin A, and tacrine.

Examples of the NMDA receptor antagonist include memantine.

Examples of the tau protein remover and production inhibitor include tau protein vaccine, tau protein removing antibody, tau protein modification inhibitor, tau protein coagulation inhibitor, and tau proteolysis promoter.

Examples of the tau protein remover and production inhibitor include TRx-237, TPI-287, ABBV-8E12, RG-6100, AADvac1, RO7105705, PTI-80, JNJ-63733657, UCB-0107, BIIB-076, MC-1, ACI-35, and AZP-2006.

Examples of the therapeutic drug for Parkinson's disease include levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.

Examples of the therapeutic drug for multiple sclerosis include steroid, interferon β, glatirameracetate, fingolimod, natalizumab, MN-166, siponimod, laquinimod, and masitinib.

The above-mentioned therapeutic drugs may be used in appropriate combinations according to the symptoms of the patients.

### 6. Therapeutic drug of the present invention

The above-mentioned therapeutic drugs for tauopathy and dementia-related diseases that can be used vary depending on the severity of the symptoms (mild, moderate, severe etc.). Therefore, the therapeutic drug to be administered to the test animal may be determined using the determined results of the method for assisting in the determination of the degree of progression of tauopathy and dementia-related diseases of the present invention described above.

Therefore, the present invention provides a therapeutic drug for tauopathy and dementia-related diseases that is used for a test animal (patient) whose degree of progression of tauopathy and dementia-related diseases has been determined.

As mentioned above, the amount of S38AA short fragment markedly increases in the blood of the patients of tauopathy and dementia-related diseases. Thus, the present invention provides a therapeutic or prophylactic drug for Alzheimer's disease that contains, as an active ingredient, a medicament that decreases the amount of S38AA short fragment in the body of patients with tauopathy and dementia-related diseases or one (human) who may be affected with tauopathy or a dementia-related disease, or a medicament that inhibits production of S38AA short fragment in the body of patients with tauopathy and dementia-related diseases or one (human) who may be affected with tauopathy or a dementia-related disease. As used herein, the amount of S38AA short fragment in the body of a patient or one who may be affected is, for example, the amount of S38AA short fragment contained in a biological tissue or body fluid of the patient or one who may be affected. Specifically, blood, cerebrospinal fluid, urine, saliva, lacrimal fluid and the like can be mentioned.

The medicament that decreases the amount of S38AA short fragment is, for example, a neutralizing antibody, and it can decrease free S38AA short fragment in the body by binding to the S38AA short fragment in the body of a patient or one who may be affected. The medicament that inhibits production of S38AA short fragment is, for example, an inhibitor of enzyme that produces 38AA short fragment by cleaving S38AA long fragment, and each of them can be obtained by methods known to those skilled in the art.

### 7. Method for selecting candidate substance for therapeutic or prophylactic drug for tauopathy and dementia-related diseases

The present invention provides a method for selecting a candidate substance for a therapeutic or prophylactic drug for tauopathy and dementia-related diseases by using whether or not a test substance removes S38AA short fragment or whether or not it inhibits the production of S38AA short fragment as an index, and a substance obtained by the method. In the selection method of the present invention, a substance that decreases the amount of an S38AA short fragment in blood, or down-regulates the production of an S38AA short fragment is selected as a candidate substance for a therapeutic or prophylactic drug for tauopathy and dementia-related diseases.

The test substance to be subjected to the selection method of the present invention may be any known or novel compound. Examples thereof include nucleic acid, carbohydrate, lipid, protein, peptide, organic low-molecular-weight compound, compound library produced using a combinatorial chemistry technique, random peptide library, natural component derived from microorganism, animals and plants, marine organism etc., and the like.

For example, the selection method of the present invention may include:
(i) a step of contacting a test substance with a cell permitting measurement of production of a S38AA short fragment;
(ii) a step of measuring the production amount of the S38AA short fragment in the cell contacted with the test substance, and comparing the production amount with that of the S38AA short fragment in a control cell free of contact with the test substance; and (iii) a step of selecting a test substance that down-regulates the production amount of the S38AA short fragment as a candidate substance for a therapeutic or prophylactic drug for tauopathy and dementia-related diseases, based on the comparison results of the above-mentioned (ii).

In addition, the selection method of the present invention may include:
(i) a step of contacting a test substance with an enzyme that forms an S38AA short fragment; (ii) a step of measuring the production amount of the S38AA short fragment in the enzyme contacted with the test substance, and comparing the production amount with that of the S38AA short fragment in a control enzyme free of contact with the test substance; and (iii) a step of selecting a test substance that down-regulates the production amount of the S38AA short fragment as a candidate substance for a therapeutic or prophylactic drug for tauopathy and dementia-related diseases, based on the comparison results of the above-mentioned (ii). The above-mentioned enzyme substrate is not particularly limited as long as it produces S38AA short fragment and, for example, S38AA, S38AA long fragment, S38AA fragment and the like can be mentioned.

Furthermore, for example, (i) a step of contacting a test substance with a S38AA short fragment, (ii) a step of measuring the amount of free S38AA short fragment remaining after contact with the test substance, and comparing the amount with that of the free S38AA short fragment when the free S38AA short fragment is not contacted with the test substance, and (iii) a step of selecting a test substance that down-regulates the amount of the free S38AA short fragment by binding to S38AA short fragment as a candidate substance for a therapeutic or prophylactic drug for tauopathy and dementia-related diseases, based on the comparison results of the above-mentioned (ii) may be included. The method may be performed by adding a test substance to a system containing an enzyme and a substrate thereof that produce the above-mentioned S38AA short fragment, or by adding a test substance to a system containing S38AA short fragment prepared in advance. Alternatively, in (i) a step of contacting a test substance with a S38AA short fragment, the S38AA short fragment may form sediments (e.g., immunoprecipitation, etc.) due to the contact.

The "cell" to be used for the selection method of the present invention means a cell permitting evaluation of the production level of the measurement target, an S38AA short fragment. Examples of the cell include a cell capable of naturally producing the S38AA short fragment of the measurement target, an S38AA-expressing cell capable of producing an S38AA short fragment by stimulation, and a genetically engineered cell to be able to produce an S38AA short fragment.

The cell capable of naturally producing an S38AA short fragment, is not particularly limited and, as such cell, a primary cultured cell of a mammal (for example, human, mouse etc.), a cell line induced from said primary cultured cell and the like can be used. S38AA is known to be expressed in U251 cell and SHSY-5Y cell, and also expressed in BE(2)-C cell and SK-N-MC cell. In addition, a genetically engineered cell overexpressing S38AA or labeled S38AA with FLAG tag etc., and the like can also be produced using a known technique. By culture, S38AA is cleaved from the S38AA expressing cell and the S38AA short fragment is liberated. When the amount of the produced S38AA short fragment is small, the production of the S38AA short fragment can be measured by cultivating, as appropriate, under conditions easily causing the cleavage of S38AA. Examples of the conditions easily causing the cleavage of S38AA include cultivating in a glucose depletion medium or a medium containing a substance known to physiologically stimulate the brain. Specific examples of such substance include cytokines such as TNFα, interferon-y, interleukin-1, interleukin-6 and the like, amyloid beta or aggregate thereof and the like.

The test substance and the cell permitting measurement of the production of an S38AA short fragment are contacted in a culture medium. The culture medium is appropriately selected according to the cell permitting measurement of the production of an S38AA short fragment. Examples thereof include minimum essential medium (MEM), Dulbecco's modified Eagle medium (DMEM), containing about 5 - 20% of fetal bovine serum, and the like. The culture conditions are appropriately determined in the same manner. For example, the pH of the medium is about 6 - about 8, the culture temperature is generally about 30 - about 40°C, and the culture time is about 0.1 - about 72 hr.

The contact between the test substance and an enzyme that forms S38AA short fragment is carried out in a reaction system containing the enzyme and a substrate thereof. The enzyme concentration, substrate concentration, pH, temperature, and the like of the reaction system, and enzyme substrate, reaction time and the like can be set as appropriate.

As used herein, as the enzyme that produces S38AA short fragment, a solution containing the enzyme can be used. Examples of the solution include body fluid (plasma, etc.), organ or tissue extract, cell extract, and the like that can form S38AA short fragment.

The production amount of the S38AA short fragment can be measured by measuring the amount of the S38AA short fragment liberated in the cell culture supernatant or in the reaction system according to the method described in the item of (5. Method of the present invention).

The production amount can be preferably compared based on the presence or absence of a significant difference. The production amount of an S38AA short fragment in the control cell or enzyme free of contact with the test substance may be measured before or simultaneously with the measurement of the production amount of the S38AA short fragment in the cell or enzyme contacted with the test substance.

The substance obtained by the selection method of the present invention is useful as a candidate substance for the development of a new therapeutic or preventive drug for tauopathy and dementia-related diseases.

### [Example]

The present invention is explained in detail in the following by referring to Examples; however, the present invention is not limited thereto.

### Reference Example 1: Identification of N-terminal cleavage site of S38AA long fragment in human plasma

After separating plasma proteins derived from AD patients by polyacrylamide gel electrophoresis (SDS-PAGE), gel fragments containing S38AA fragments were cut out and, after in-gel digestion with trypsin, and analyzed by liquid chromatography-mass spectrometry (LC-MS/MS). The measurement data was subjected to MASCOT database search, and the N-terminal sequence of the S38AA fragment was determined.

Specifically, a mixed sample of the plasma of some AD patients was applied to 4-12% Bis-Tris Gel (invitrogen), and protein was separated by SDS-PAGE (25 mA, 110 min, MOPS buffer). After staining the gel with Coomassie Brilliant Blue staining, the band seen at 80-100 kDa was cut out and applied to the digestion process.

The cut gel fragments were placed in a 96-well microplate, acetonitrile was added, the mixture was centrifuged under reduced pressure, 10 mM DTT/100 mM NH₄HCO₃ was added, and incubation was performed. After removing the solvent, 55 mM ICH₂CONH₂/100 mM NH₄HCO₃ was added and the mixture was incubated. After removing the solvent, 100 mM NH₄HCO₃ was added, the gel was centrifuged under reduced pressure, 0.1% RapiGest/25 mM NH₄HCO₃ was added, and incubation was performed. Then, the mixture was centrifugally dried under reduced pressure, an enzyme solution (50 mM NH₄HCO₃, 12.5 ng/µL trypsin) was added, and enzyme digestion was performed. After the reaction, the peptide solution was transferred to another 96-well microplate, a mixed solvent of acetonitrile:milliQ:trifluoroacetic acid (TFA)=500:500:1 was added to the gel, and the obtained peptide extract was concentrated under reduced pressure. TFA was added thereto, and the mixture was concentrated under reduced pressure to give a sample for MS analysis.

The obtained sample was analyzed by LC-MS/MS. When the data obtained by the Orbitrap mass spectrometer was subjected to MASCOT database search for the amino acid sequence of S38AA protein, multiple peptide fragments were identified in the amino acid sequence of the S38AA protein (Fig. 1). Of these sequences, the peptide which is closest to the N-terminal side is the 399th to 413th amino acid sequence, and an MS/MS spectrum showing the sequence was observed (Fig. 2). This cleavage site was on the C-terminal side of the 398th serine (S). As for the cleavage of digestive enzyme trypsin, since the enzyme specifically cleaves the C-terminal of lysine (K) or arginine (R), and the cleavage reaction does not occur on the C-terminal side of serine (S) of the fragment, the cleavage having already been performed at this site was suggested. The detection of the peptide fragment revealed that the N-terminal side of the purified S38AA fragment was cleaved at the 398th to 399th S/E. The S38AA fragment identified here is referred to as S38AA long fragment.

### Reference Example 2: Identification of N-terminal cleavage site of S38AA short fragment in human plasma

After removing S38AA long fragment by immunoprecipitation method using rabbit-derived anti-S38AA polyclonal antibody that recognizes the N-terminal of S38AA Long fragment (to be also referred to as "MBL", "antibody for S38AA long fragment", or "antibody for long fragment"), remaining S38AA fragments were immunoprecipitated using mouse-derived anti-S38AA monoclonal antibody A (antibody-producing hybridoma was established using a part of the peptide of the C-terminal amino acid sequence of S38AA fragment as an immunogen, and separated and purified; antibody A), mouse-derived anti-S38AA monoclonal antibody B (antibody-producing hybridoma was established using a part of the peptide of the C-terminal amino acid sequence of S38AA fragment as an immunogen, and separated and purified; antibody B), or mouse-derived anti-S38AA monoclonal antibody C (antibody-producing hybridoma was established using recombinant protein of S38AA long fragment in Escherichia coli as immunogen, and separated and purified; antibody C), fractions thereof were purified by reverse-phase column, digested with trypsin, analyzed by LC-MS/MS, and the N-terminal sequence of the S38AA fragment was determined.

Specifically, to a pooled mixture of the plasma of AD patients was added PBS containing a protease inhibitor (complete Tablets Mini, Roche), and Protein G Mag Sepharose Xtra (bead, GE HEALTHCARE) was further added and mixed to remove endogenous immunoglobulins. Antibody for long fragment was added to the supernatant after removal of beads, and antigen-antibody reaction was performed. After mixing, beads were newly added, and recovery protein containing antibody for long fragment and S38AA long fragment adsorbed to the antibody was removed. To the supernatant after removal of beads was added the aforementioned antibody A, B or C, and antigen-antibody reaction was performed. After mixing, beads were newly added, and the beads were collected to obtain the antibody and the S38AA fragment adsorbed on the antibody. An 8M urea/1% TFA solution was added to the beads recovered by immunoprecipitation, and the S38AA fragment was eluted. The obtained solution containing the S38AA fragment was concentrated, and the total amount was separated by reverse-phase column (ZORBAX 300SB-C3, 4.6x150 mm with guard column, Agilent). The separation conditions are as shown in Table 1 below. The obtained each fraction was measured by sandwich ELISA using two antibodies recognizing the C-terminal side region of the S38AA fragment, fractions containing S38AA fragment were digested with an enzyme solution (1M NH₄HCO₃, 10 mM CaCl₂, 1 ng/µL trypsin), and the obtained peptide was concentrated with GL-tip SDB and GL-Tip GC (GL Sciences).

The obtained peptide was analyzed by LC-MS/MS. When the data obtained by the Q-Exactive HF mass spectrometer was subjected to MASCOT database search for the amino acid sequence of S38AA protein, S38AA was identified with the highest score and multiple peptide fragments were identified (Fig. 3). Of these sequences, the peptide which is the closest to the N-terminal side is the 617th to 627th sequence, and an MS/MS spectrum showing the sequence was observed (Fig. 4). This cleavage site was on the C-terminal side of the 616th asparagine (N). As for the cleavage of digestive enzyme trypsin, since the enzyme specifically cleaves the C-terminal of lysine (K) or arginine (R), and the cleavage reaction does not occur on the C-terminal side of asparagine (N) of the fragment, the cleavage having already been performed at this site was shown. The detection of the peptide fragment revealed that the purified S38AA fragment was cleaved at the 617th to 618th N/G. The S38AA fragment identified here is referred to as S38AA short fragment.

### [Table 1]

**Table 1 reverse-phase column purification separation conditions**

| Flow 1 ml/min | | | Temp 70 |
|---|---|---|---|
| | A: 0.1% TFA/H₂O | | |
| | B: 0.1% TFA/acetonitrile | | |

| gradient conditions | | min | %B |
|---|---|---|---|
| | | 0 | 20 |
| | | 40 | 80 |
| | | 41 | 95 |
| | | 46 | 95 |
| | | 47 | 20 |
| | | 57 | 20 |

### Reference Example 3: Identification of C-terminal cleavage site of S38AA long fragment in human plasma

S38AA long fragment was separated by the antibody column purification method using "polyclonal antibody for rabbit-derived S38AA long fragment (antibody for long fragment)" used in Reference Example 2, gel fragments containing S38AA long fragments were cut out and, after in-gel digestion with trypsin, analyzed by LC-MS/MS. The measurement data was subjected to MASCOT database search, and the C-terminal fragment sequence of the S38AA long fragment was determined.

Specifically, to a mixed sample of the plasma of some AD patients was added 50 mM Tris-HCl/0.05% Tween-20 (pH 7.4), and the mixture was applied to anion exchange column purification (Hitrap Q FF, GE HEALTHCARE). Successively, it was eluted with 50 mM phosphoric acid/0.05% Tween-20/500 mM NaCl (pH 7.4). The eluted fraction was applied to a column (HiTrap NHS-activated HP column, GE HEALTHCARE) bound with antibody for long fragment. The column was washed with PBS-T, and eluted with 0.1 M Glycine-HCl/0.05% Tween-20 (pH2.7). The eluate was immediately returned to neutral with 1M Tris-HCl (pH9.0). The obtained sample was applied to HiTrap Q FF column (GE HEALTHCARE) equilibrated in advance with 50 mM Tris-HCl (pH 7.4), and the surfactant was removed. The sample was concentrated by centrifugation under reduced pressure, 50 mM DTT/LDS buffer was added, and the mixture was heated. The total amount of the sample was applied to 4-12% Bis-Tris Gel (Invitrogen), and protein was separated by SDS-PAGE (50 mA, 90 min, MOPS buffer). After staining the gel with Sypro Ruby (pierce), the band seen at 80-100 kDa was cut out and applied to the digestion process.

The cut gels were placed in a 96-well microplate, acetonitrile was added, and sonication was performed. Thereafter, the mixture was centrifugally dried under reduced pressure, 10 mM DTT/25 mM NH₄HCO₃ was added, and incubation was performed. After removing the solvent, 55 mM ICH₂CONH₂/25 mM NH₄HCO₃ was added, and incubation was performed under shading. After removing the solvent, 50 mM NH₄HCO₃ was added, the mixture was incubated, acetonitrile was added, and sonication was performed. After removing the solvent, the gel was centrifugally dried under reduced pressure, 0.1% RapiGest/25 mM NH₄HCO₃ was added, and incubation was performed. Thereafter, the mixture was centrifugally dried under reduced pressure, incubation was performed, an enzyme solution (50 mM NH₄HCO₃, 5 ng/uL trypsin) was added, and the mixture was incubated. After removing the solvent, 50 mM NH₄HCO₃ was added, the mixture was incubated, and enzyme digestion was performed. After the reaction, the peptide solution was transferred to another 96-well microplate, a mixed solvent of acetonitrile:milliQ:TFA=500:500:1 was added to the gel, and sonication was performed. This operation was repeated again, and the obtained peptide extract was concentrated under reduced pressure to give a sample for MS analysis.

The obtained peptide was analyzed by LC-MS/MS. When the data obtained by the Orbitrap mass spectrometer was subjected to MASCOT database search for the amino acid sequence of S38AA protein, multiple peptide fragments were identified in the amino acid sequence of the S38AA protein (Fig. 5). Of these sequences, the peptide which is the closest to the C-terminal side is the 1040th to 1049th sequence, and an MS/MS spectrum showing the sequence was observed (Fig. 6). This cleavage site was on the C-terminal side of the 1049th leucine (L). As for the cleavage of digestive enzyme trypsin, since the enzyme specifically cleaves the C-terminal of lysine (K) or arginine (R), and the cleavage reaction does not occur on the C-terminal side of leucine (L) of the fragment, the cleavage having already been performed at this site was suggested. The detection of the peptide fragment revealed that the C-terminal of S38AA long fragment was cleaved at the 1049th to 1050th L/R. Since the S38AA short fragment had the same reactivity to the "antibody (antibody A, antibody B, or antibody C) that recognizes the C-terminal of the S38AA long fragment" used in Reference Example 2, it was considered that the C-terminal of the S38AA short fragment was also cleaved at the same site.

### Reference Example 4: Production of recombinant proteins of S38AA long fragment and S38AA short fragment in Escherichia coli and measurement of them by ELISA

Recombinant proteins in Escherichia coli of S38AA long fragment and S38AA short fragment were produced. Using them as the reference standard, Long ELISA that quantifies only S38AA long fragment and Total ELISA that quantifies both fragments were constructed.

Specifically, a transformant of Escherichia coli BL21(DE3) into which plasmid DNAs of S38AA long fragment sequence (399-1049 amino acid sequence) and S38AA short fragment sequence (617-1049 amino acid sequence) were introduced was produced. Shaking culture was continued, and the cells were harvested by centrifugation. A protein extraction reagent B-PER (Thermo Fisher Scientific) was suspended in a small amount of bacterial bodies, the centrifugation supernatant was electrophoresed by SDS-PAGE, and the expression of the targeted protein was confirmed. The bacterial bodies were suspended in buffer A (20 mM Tris HCl pH 8.0, 200 mM NaCl, 10% glycerol, 20 mM imidazole), protease inhibitor complete EDTA-free (Roche), and nuclease Benzonase to disrupt the bacterial bodies, and the suspension was centrifuged. Using a 0.22 µm filter, residual bacterial bodies were removed from the centrifugation supernatant, and Ni affinity purification was performed using HisTrap HP. Elution fractions of the object protein were brought together and used as the reference standard. The protein concentration of the reference standard was measured to fine 1.4 mg/mL (long fragment), and 1.6 mg/mL (short fragment), respectively.

Sandwich ELISA (Long ELISA) by "antibody that recognizes the N-terminal side region of S38AA long fragment (antibody for long fragment used in Reference Example 2)", "antibody A that recognizes C-terminal side region", "antibody B", or "antibody C", and sandwich ELISA (Total ELISA) by two antibodies selected from "antibody A", "antibody B" and "antibody C" that recognizes the C-terminal side region common to both S38AA long fragment and S38AA short fragment were generated. As a reference standard for quantification, the above-mentioned recombinant protein in Escherichia coli (standard protein) was prepared and measured by the both ELISAs. The sample was applied to a plate on which each antibody was immobilized, and incubated. After washing 3 times, each HRP-labeled antibody was added and incubated. After washing three times, a 3% 3,3',5,5'-Tetramethylbenzidine (TMB) solution was added, and the mixture was incubated under shading. Finally, 8% sulfuric acid was added to discontinue the reaction, and the absorbance (O.D.) at 450 nm was measured. As a result, a standard protein concentration-dependent reaction was observed, and a good calibration curve was obtained (Fig. 7). In Long ELISA, no reaction to S38AA short fragment standard protein was observed.

### Example 1: Quantification of S38AA long fragment and S38AA short fragment in human plasma (Total ELISA-Long ELISA subtraction method)

To quantify S38AA long fragment and S38AA short fragment contained in human plasma, quantification by ELISA using a subtraction method was performed.

Specifically, the amount of S38AA short fragment contained in each plasma derived from 5 healthy subjects and 5 PSP patients, 5 CBD patients, 5 MSA patients, 5 ALS patients, 5 PDD patients, and 5 MS patients was quantified using the aforementioned Total ELISA and Long ELISA. Quantification was performed according to the method described in Reference Example 4. The calibration curve of each ELISA was created by the measurement of S38AA long fragment standard protein. The amount of S38AA short fragment was calculated by subtracting the quantified value of Long ELISA from the quantified value of the Total ELISA. The quantified values of S38AA long fragment and S38AA short fragment in each sample were obtained.

As a result of the measurement, it was clarified that the amount of S38AA long fragment and the amount of S38AA short fragment were different between the "healthy human" and the "PSP patients, CBD patients, MSA patients, PDD patients, and MS patients". Therefore, it was clarified that the patients can be determined/screened with high sensitivity by measuring the amount of the S38AA long fragment or the amount of the S38AA short fragment (Fig. 8).

Particularly, it was clarified that the amount of S38AA short fragment was significantly different between the "healthy human" and the "PSP patients, CBD patients, MSA patients, PDD patients, and MS patients". As for the disease, a large difference was observed particularly in the "PSP patients, PDD patients, and MS patients".

### Example 2: Quantification of S38AA long fragment and S38AA short fragment in human plasma (Total ELISA-Long ELISA subtraction method)

To quantify S38AA long fragment and S38AA short fragment contained in human plasma, quantification by ELISA using a subtraction method was performed.

Specifically, the amount of S38AA short fragment contained in each plasma derived from 5 healthy subjects and 5 PSP patients, 5 DLB patients, 10 FTD patients, 10 MCI patients, 5 PD patients, and 5 VaD patients was quantified using the aforementioned Total ELISA and Long ELISA. Quantification was performed according to the method described in Reference Example 4. The calibration curve of each ELISA was created by the measurement of S38AA long fragment standard protein. The amount of S38AA short fragment was calculated by subtracting the quantified value of Long ELISA from the quantified value of the Total ELISA. The quantified values of S38AA long fragment and S38AA short fragment in each sample were obtained.

As a result of the measurement, it was clarified that the amount of S38AA long fragment and the amount of S38AA short fragment were different between the "healthy human" and the "DLB patients, FTD patients, MCI patients, PD patients, and VaD patients". Therefore, it was clarified that the patients can be determined/selected with high sensitivity by measuring the amount of the S38AA long fragment or the amount of the S38AA short fragment (Fig. 9).

Particularly, it was clarified that the amount of S38AA short fragment was significantly different between the "healthy human" and the "DLB patients, FTD patients, MCI patients, PD patients, and VaD patients". As for the disease, a large difference was observed particularly from the "VaD patients".

### [Industrial Applicability]

The present invention is useful for the diagnosis and treatment and the like of tauopathy and dementia-related diseases.

This application is based on a patent application No. 2020-018249 filed in Japan (filing date: February 5, 2020), the contents of which are incorporated in full herein.

## Claims

1. A kit for use in determining tauopathy or a dementia-related disease (excluding Alzheimer's disease), comprising an antibody that recognizes a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1.

2. The kit according to claim 1, wherein the polypeptide consists of the amino acid sequence shown in SEQ ID NO: 1.

3. The kit according to claim 1 or 2, wherein the antibody further recognizes the polypeptide of SEQ ID NO: 2.

4. The kit according to any one of claims 1 to 3, wherein
the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

5. The kit according to any one of claims 1 to 4, wherein
the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

6. The kit according to any one of claims 1 to 5, wherein
the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

7. The kit according to any one of claims 1 to 6, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

8. The kit according to any one of claims 1 to 7, further comprising a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1.

9. The kit according to any one of claims 1 to 8, wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

10. An agent for determining tauopathy or a dementia-related disease (excluding Alzheimer's disease), comprising an antibody capable of measuring an amount of a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1.

11. The agent according to claim 10, wherein the polypeptide consists of the amino acid sequence shown in SEQ ID NO: 1.

12. The agent according to claim 10 or 11, wherein the antibody further recognizes the polypeptide of SEQ ID NO: 2.

13. The agent according to any one of claims 10 to 12,
wherein the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

14. The agent according to any one of claims 10 to 13,
wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

15. The agent according to any one of claims 10 to 14,
wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

16. The agent according to any one of claims 10 to 15,
wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

17. The agent according to any one of claims 10 to 16, further comprising a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1.

18. The agent according to any one of claims 10 to 17,
wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

19. Use of an antibody that recognizes a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1 in the manufacture of an agent for determining tauopathy or a dementia-related disease (excluding Alzheimer's disease).

20. The use according to claim 19, wherein the polypeptide consists of the amino acid sequence shown in SEQ ID NO: 1.

21. The use according to claim 19 or 20, wherein the antibody further recognizes the polypeptide of SEQ ID NO: 2.

22. The use according to any one of claims 19 to 21, wherein
the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

23. The use according to any one of claims 19 to 22, wherein
the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

24. The use according to any one of claims 19 to 23, wherein
the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

25. The use according to any one of claims 19 to 24, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

26. The kit according to any one of claims 19 to 25, further comprising a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1.

27. The use according to any one of claims 19 to 26, wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

28. A method for determining whether a test animal is affected with tauopathy or a dementia-related disease at present or may be affected with tauopathy or a dementia-related disease in the future, comprising detecting a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1 in a sample collected from the test animal, wherein the tauopathy and dementia-related disease do not include Alzheimer's disease.

29. The method according to claim 28, comprising the following steps (i) to (iii):
(i) a step of quantifying the polypeptide in a sample collected from a test animal,
(ii) a step of comparing the amount of the polypeptide quantified in (i) with the amount of the polypeptide in a sample collected from a healthy animal (hereinafter to be referred to as control value), and
(iii) a step of determining based on the results of (ii) that the test animal may be affected with tauopathy or a dementia-related disease at present or that the animal may be affected with tauopathy or a dementia-related disease in the future, when the amount of the polypeptide quantified in (i) is higher than the control value.

30. The method according to claim 29, wherein the amount of the polypeptide quantified in (i) is not less than 1.1 times of the control value.

31. The method according to claim 28, comprising the following steps (i) and (ii):
(i) a step of quantifying the polypeptide in a sample collected from a test animal,
(ii) a step of determining that the test animal may be affected with tauopathy or a dementia-related disease at present or that the animal may be affected with tauopathy or a dementia-related disease in the future when the amount of the polypeptide quantified in (i) is higher than the cutoff value.

32. The method according to claim 31, wherein the cutoff value is 45 - 85 units.

33. The method according to claim 31, wherein the cutoff value is 45 - 85 ng/mL.

34. The method according to any one of claims 28 to 33, wherein the test animal is a human.

35. The method according to any one of claims 28 to 34, wherein the sample is blood, cerebrospinal fluid, saliva, lacrimal fluid, or urine.

36. The method according to any one of claims 28 to 35, further comprising detecting other one or more tauopathy and dementia-related disease diagnosis markers.

37. The method according to any one of claims 28 to 36, wherein the polypeptide consists of the amino acid sequence shown in SEQ ID NO: 1.

38. The method according to any one of claims 28 to 37, wherein the polypeptide is detected using an antibody.

39. The method according to claim 38, wherein the antibody further recognizes the polypeptide of SEQ ID NO: 2.

40. The method according to any one of claims 38 and 39,
wherein the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

41. The method according to any one of claims 38 to 40,
wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

42. The method according to any one of claims 38 to 41, wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

43. The method according to any one of claims 38 to 42, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

44. The method according to any one of claims 28 to 43,
wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

45. A method for determining the degree of progression of tauopathy or a dementia-related disease (excluding Alzheimer's disease), comprising detecting a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in a sample collected from a test animal.

46. The method according to claim 45, comprising the following steps (i) to (iii):
(i) a step of quantifying the polypeptide in a sample collected from a test animal that is or may be affected with tauopathy or a dementia-related disease,
(ii) a step of comparing the amount of the polypeptide quantified in (i) with the amount of the polypeptide in a sample collected from an animal affected with tauopathy or the dementia-related disease at a specific degree of progression (hereinafter control value), and
(iii) a step of determining, based on the results of (ii), that the degree of progression of the tauopathy or dementia-related disease of the test animal is higher than that of an animal affected with the disease as a control when the amount of the polypeptide quantified in (i) is higher than the control value, and that the degree of progression of the tauopathy or dementia-related disease of the test animal is lower than that of an animal affected with the disease as a control when the amount is smaller than the control value.

47. The method according to claim 45, comprising the following steps (i) to (iii):
(i) a step of quantifying the polypeptide in a sample collected from a test animal that is or may be affected with tauopathy or a dementia-related disease,
(ii) a step of comparing the amount of the polypeptide quantified in (i) with the amount of the polypeptide in a sample collected in the past from the test animal (hereinafter control value), and
(iii) a step of determining, based on the results of (ii), that the tauopathy or dementia-related disease of the test animal is progressing when the amount of the polypeptide quantified in (i) is higher than the control value, and that the tauopathy or dementia-related disease of the test animal was improved when the amount is smaller than the control value.

48. The method according to any one of claims 46 and 47,
wherein the tauopathy or dementia-related disease of the test animal is determined to be progressing when the amount of the polypeptide quantified in (i) is not less than 1.1 times of the control value.

49. The method according to any one of claims 46 and 47,
wherein the tauopathy or dementia-related disease of the test animal is determined to be improved when the amount of the polypeptide quantified in (i) is not more than 0.9 times of the control value.

50. The method according to claim 45, comprising the following steps (i) to (iii):
(i) a step of quantifying the polypeptide in a sample collected from a test animal,
(ii) a step of determining that tauopathy or a dementia-related disease of the test animal is exacerbated when the amount of the polypeptide quantified in (i) is higher than the cutoff value.

51. The method according to any one of claims 45 to 50, wherein the test animal is a human.

52. The method according to any one of claims 45 to 51, wherein the sample is blood, cerebrospinal fluid, saliva, lacrimal fluid, or urine.

53. The method according to any one of claims 45 to 52, further comprising detecting other one or more tauopathy and dementia-related disease diagnosis markers.

54. The method according to any one of claims 45 to 53, wherein the polypeptide consists of the amino acid sequence shown in SEQ ID NO: 1.

55. The method according to any one of claims 45 to 54, wherein the polypeptide is detected using an antibody.

56. The method according to claim 55, wherein the antibody further recognizes the polypeptide of SEQ ID NO: 2.

57. The method according to claim 55 or 56, wherein the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

58. The method according to any one of claims 55 to 57,
wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

59. The method according to any one of claims 55 to 58,
wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

60. The method according to any one of claims 55 to 59, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

61. The method according to any one of claims 45 to 60,
wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

62. A method for treating or preventing tauopathy or a dementia-related disease, comprising detecting a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in a sample collected from a test animal, and administering a therapeutic drug for tauopathy and dementia-related diseases to the test animal, wherein the tauopathy and dementia-related diseases do not include Alzheimer's disease.

63. The method according to claim 62, comprising the following steps (i) to (iv):
(i) a step of quantifying the polypeptide according to any one of claims 1 and 2 in a sample collected from a test animal,
(ii) a step of comparing the amount of the polypeptide quantified in (i) with the amount of the polypeptide in a sample collected from a healthy animal (hereinafter to be referred to as control value),
(iii) a step of determining, based on the results of (ii), that the test animal is or may be affected with tauopathy or a dementia-related disease at present, or may be affected with tauopathy or a dementia-related disease in the future when the amount of the polypeptide quantified in (i) is higher than the control value, and
(iv) a step of administering, based on the results of (iii), a therapeutic or prophylactic drug for tauopathy and dementia-related diseases to a test animal determined to be affected or possibly affected with tauopathy or a dementia-related disease at present, or possibly affected with tauopathy or a dementia-related disease in the future.

64. The method according to claim 63, wherein the amount of the polypeptide quantified in (i) is not less than 1.1 times of the control value.

65. The method according to claim 62, comprising the following steps (i) and (ii):
(i) a step of quantifying the polypeptide in a sample collected from a test animal,
(ii) a step of determining that the test animal may be affected with tauopathy or a dementia-related disease at present or that the animal may be affected with tauopathy or a dementia-related disease in the future when the amount of the polypeptide quantified in (i) is higher than the cutoff value.

66. The method according to claim 65, wherein the cutoff value is 45 - 85 units.

67. The method according to claim 65, wherein the cutoff value is 45 - 85 ng/mL.

68. The method according to any one of claims 62 to 67, wherein the test animal is a human.

69. The method according to any one of claims 62 to 68, wherein the sample is blood, cerebrospinal fluid, saliva, lacrimal fluid, or urine.

70. The method according to any one of claims 62 to 69, further comprising detecting other one or more tauopathy and dementia-related disease diagnosis markers.

71. The method according to any one of claims 62 to 70, wherein the polypeptide consists of the amino acid sequence shown in SEQ ID NO: 1.

72. The method according to any one of claims 62 to 71, wherein the polypeptide is detected using an antibody.

73. The method according to claim 72, wherein the antibody further recognizes the polypeptide of SEQ ID NO: 2.

74. The method according to any one of claims 72 to 73,
wherein the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

75. The method according to any one of claims 72 to 74,
wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

76. The method according to any one of claims 72 to 75,
wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

77. The method according to any one of claims 72 to 76, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

78. The method according to any one of claims 62 to 77,
wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

79. The method according to any one of claims 62 to 78,
wherein the therapeutic drug for tauopathy or a dementia-related disease is selected from the group consisting of cholinesterase inhibitor, NMDA receptor antagonist, tau protein remover and production inhibitor, therapeutic drug for Parkinson's disease, and therapeutic drug for multiple sclerosis.

80. The method according to claim 79, wherein the cholinesterase inhibitor is at least one selected from the group consisting of donepezil, galanthamine, rivastigmine, Huperzine A, and tacrine.

81. The method according to claim 79, wherein the NMDA receptor antagonist is memantine.

82. The method according to claim 79, wherein the tau protein remover and production inhibitor are at least one selected from the group consisting of tau protein vaccine, tau protein removing antibody, tau protein modifying inhibitor, tau protein coagulation inhibitor, and tau proteolysis promoter.

83. The method according to claim 82, wherein the tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.

84. The method according to claim 79, wherein the therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.

85. The method according to claim 79, wherein the therapeutic drug for multiple sclerosis is at least one selected from the group consisting of steroid, interferon β, glatirameracetate, fingolimod, natalizumab, MN-166, siponimod, laquinimod, and masitinib.

86. A method for administering a medicine for the treatment or prophylaxis of tauopathy or a dementia-related disease, comprising quantifying a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in a sample collected from a test animal, selecting a therapeutic or prophylactic drug for tauopathy or a dementia-related disease, and administering the therapeutic drug for tauopathy or a dementia-related disease to the test animal,
wherein the tauopathy and dementia-related disease do not include Alzheimer's disease.

87. The method according to claim 86, comprising
(i) a step of quantifying the polypeptide in a sample collected from a test animal that is or may be affected with tauopathy or a dementia-related disease at present,
(ii) a step of comparing the amount of the polypeptide quantified in (i) with the amount of the polypeptide in a sample collected in the past from the test animal (hereinafter control value),
(iii) a step of determining, based on the results of (ii), that the tauopathy or dementia-related disease of the test animal is progressing when the amount of the polypeptide quantified in (i) is higher than the control value, and that the tauopathy or dementia-related disease of the test animal was improved when the amount is smaller than the control value,
(iv) a step of selecting a therapeutic or prophylactic drug for tauopathy or a dementia-related disease based on the results of (iii), and
(v) a step of administering the therapeutic drug for tauopathy or a dementia-related disease selected in (iv) to a test animal.

88. The method according to claim 87, wherein the tauopathy or a dementia-related disease in the test animal is determined to be progressing when the amount of the polypeptide quantified in (i) is not less than 1.1 times the control value.

89. The method according to claim 87, wherein the tauopathy or a dementia-related disease in the test animal is determined to be improving when the amount of the polypeptide quantified in (i) is not more than 0.9 times the control value.

90. The method according to any one of claims 86 to 89, wherein the test animal is a human.

91. The method according to any one of claims 86 to 90, wherein the sample is blood, cerebrospinal fluid, saliva, lacrimal fluid or urine.

92. The method according to any one of claims 86 to 91, further comprising detecting other one or more tauopathy and dementia-related disease diagnosis markers.

93. The method according to any one of claims 86 to 92, wherein the polypeptide consists of the amino acid sequence shown in SEQ ID NO: 1.

94. The method according to any one of claims 86 to 93, wherein the polypeptide is detected using an antibody.

95. The method according to claim 94, wherein the antibody further recognizes the polypeptide of SEQ ID NO: 2.

96. The method according to claim 94 or 95, wherein the
antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

97. The method according to any one of claims 94 to 96,
wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

98. The method according to any one of claims 94 to 97, wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16,SEQ ID NO: 18, SEQ ID NO: 20, or SEQ ID NO: 22, and the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

99. The method according to any one of claims 94 to 98, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

100. The method according to any one of claims 86 to 99,
wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

101. The method according to any one of claims 86 to 100,
wherein the therapeutic drug for tauopathy or a dementia-related disease is selected from the group consisting of cholinesterase inhibitor, NMDA receptor antagonist, and tau protein remover and production inhibitor.

102. The method according to claim 101, wherein the
cholinesterase inhibitor is at least one selected from the group consisting of donepezil, galanthamine, rivastigmine, Huperzine A, and tacrine.

103. The method according to claim 101, wherein the NMDA receptor antagonist is memantine.

104. The method according to claim 101, wherein the tau protein remover and production inhibitor are at least one selected from the group consisting of tau protein vaccine, tau protein removing antibody, tau protein modifying inhibitor, tau protein coagulation inhibitor, and tau proteolysis promoter.

105. The method according to claim 104, wherein the tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.

106. The method according to claim 101, wherein the therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.

107. The method according to claim 101, wherein the therapeutic drug for multiple sclerosis is at least one selected from the group consisting of steroid, interferon β, glatirameracetate, fingolimod, natalizumab, MN-166, siponimod, laquinimod, and masitinib.

108. A therapeutic drug for tauopathy or a dementia-related disease for use for a patient with determined degree of progression of tauopathy or the dementia-related disease after quantifying a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in a sample collected from a test animal, wherein the tauopathy and dementia-related disease do not include Alzheimer's disease.

109. The therapeutic drug according to claim 108 for use for a patient whose degree of progression of tauopathy and dementia-related diseases has been determined by the following steps (i) to (iv):
(i) a step of quantifying the polypeptide in a sample collected from a test animal that is or may be affected with tauopathy or a dementia-related disease at present,
(ii) a step of comparing the amount of the polypeptide quantified in (i) with the amount of the polypeptide in a sample collected in the past from the test animal (hereinafter control value),
(iii) a step of determining, based on the results of (ii), that the tauopathy or dementia-related disease of the test animal is progressing when the amount of the polypeptide quantified in (i) is higher than the control value, and that the tauopathy or dementia-related disease of the test animal was improved when the amount is smaller than the control value, and
(iv) a step of determining, based on the results of (iii), administration of the therapeutic drug for tauopathy or a dementia-related disease.

110. The therapeutic drug according to claim 109, wherein the tauopathy or dementia-related disease of the test animal is determined to be progressing when the amount of the polypeptide quantified in (i) is not less than 1.1 times of the control value.

111. The therapeutic drug according to claim 109, wherein the tauopathy or dementia-related disease of the test animal is determined to be improved when the amount of the polypeptide quantified in (i) is not more than 0.9 times of the control value.

112. A therapeutic drug for tauopathy or a dementia-related disease for use for a patient determined to be possibly affected with tauopathy or the dementia-related disease at present or possibly affected with tauopathy or the dementia-related disease in the future:
(i) a step of quantifying the polypeptide in a sample collected from a test animal that is or may be affected with tauopathy or a dementia-related disease at present,
(ii) a step of determining that the test animal may be affected with tauopathy or a dementia-related disease at present or that the animal may be affected with tauopathy or a dementia-related disease in the future when the amount of the polypeptide quantified in (i) is higher than the cutoff value.
(iii) a step of determining, based on the results of (ii), administration of the therapeutic drug for tauopathy or a dementia-related disease, wherein the tauopathy and dementia-related disease do not include Alzheimer's disease.

113. The therapeutic drug according to claim 112, wherein the cutoff value is 45 - 85 units.

114. The therapeutic drug according to claim 112, wherein the cutoff value is 45 - 85 ng/mL.

115. The therapeutic drug according to any one of claims 108 to 114, wherein the test animal is a human.

116. The therapeutic drug according to any one of claims 108 to 115, wherein the sample is blood, cerebrospinal fluid, saliva, lacrimal fluid or urine.

117. The therapeutic drug according to any one of claims 108 to 116, further comprising detecting other one or more tauopathy and dementia-related disease diagnosis markers.

118. The therapeutic drug according to any one of claims 108 to 117, wherein the polypeptide consists of the amino acid sequence shown in SEQ ID NO: 1.

119. The therapeutic drug according to any one of claims 108 to 118, wherein the polypeptide is detected using an antibody.

120. The therapeutic drug according to claim 119, wherein the antibody further recognizes the polypeptide of SEQ ID NO: 2.

121. The therapeutic drug according to any one of claim 119 and claim 120, wherein the antibody has a heavy chain variable region having an amino acid sequence having at least 95% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 95% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

122. The therapeutic drug according to any one of claims 119 to 121, wherein the antibody has a heavy chain variable region having an amino acid sequence having at least 99% homology with SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and
a light chain variable region having an amino acid sequence having at least 99% homology with SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

123. The therapeutic drug according to any one of claims 119 to 122, wherein the amino acid sequence of the heavy chain variable region of the antibody is SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, or SEQ ID NO: 22, and the amino acid sequence of the light chain variable region of the antibody is SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, or SEQ ID NO: 23.

124. The therapeutic drug according to any one of claims 119 to 123, wherein the antibody is
(1) an antibody comprising the heavy chain variable region of SEQ ID NO: 4, and the light chain variable region of SEQ ID NO: 5,
(2) an antibody comprising the heavy chain variable region of SEQ ID NO: 6, and the light chain variable region of SEQ ID NO: 7,
(3) an antibody comprising the heavy chain variable region of SEQ ID NO: 8, and the light chain variable region of SEQ ID NO: 9,
(4) an antibody comprising the heavy chain variable region of SEQ ID NO: 10, and the light chain variable region of SEQ ID NO: 11,
(5) an antibody comprising the heavy chain variable region of SEQ ID NO: 12, and the light chain variable region of SEQ ID NO: 13,
(6) an antibody comprising the heavy chain variable region of SEQ ID NO: 14, and the light chain variable region of SEQ ID NO: 15,
(7) an antibody comprising the heavy chain variable region of SEQ ID NO: 16, and the light chain variable region of SEQ ID NO: 17,
(8) an antibody comprising the heavy chain variable region of SEQ ID NO: 18, and the light chain variable region of SEQ ID NO: 19,
(9) an antibody comprising the heavy chain variable region of SEQ ID NO: 20, and the light chain variable region of SEQ ID NO: 21, or
(10) an antibody comprising the heavy chain variable region of SEQ ID NO: 22, and the light chain variable region of SEQ ID NO: 23.

125. The therapeutic drug according to any one of claims 108 to 124, wherein the tauopathy or dementia-related disease is at least one disease selected from the group consisting of progressive supranuclear palsy (PSP), corticobasal degeneration (CBD), multiple system atrophy (MSA), pick disease (PiD), frontotemporal dementia (FTD), dementia with Lewy Bodies (DLB), vascular dementia (VaD), cognitive dysfunction associated with Parkinson's disease (PDD), and multiple sclerosis (MS).

126. The therapeutic drug according to any one of claims 108 to 125, wherein the therapeutic drug for tauopathy or a dementia-related disease is selected from the group consisting of cholinesterase inhibitor, NMDA receptor antagonist, amyloid β remover and production inhibitor, and Tau protein remover and production inhibitor.

127. The therapeutic drug according to claim 126, wherein the cholinesterase inhibitor is at least one selected from the group consisting of donepezil, galanthamine, rivastigmine, Huperzine A, and tacrine.

128. The therapeutic drug according to claim 126, wherein the NMDA receptor antagonist is memantine.

129. The therapeutic drug according to claim 126, wherein the tau protein remover and production inhibitor are at least one selected from the group consisting of tau protein vaccine, tau protein removing antibody, tau protein modifying inhibitor, tau protein coagulation inhibitor, and tau proteolysis promoter.

130. The therapeutic drug according to claim 129, wherein the tau protein remover and production inhibitor are at least one selected from the group consisting of TRx-237, TPI-287, ABBV-8E12, RG-6100, AADvac1, RO7105705, PTI-80, JNJ-63733657, UCB-0107, BIIB-076, MC-1, ACI-35, and AZP-2006.

131. The therapeutic drug according to claim 126, wherein the therapeutic drug for Parkinson's disease is at least one selected from the group consisting of levodopa, carbidopa, benserazide, selegiline, rasagiline, zonisamide, entacapone, amantadine, talipexole, pramipexole, ropinirole, rotigotine, apomorphine, cabergoline, pergolide, bromocriptine, istradefylline, trihexyphenidyl, biperiden, piroheptine, profenamine, promethazine, mexan, droxidopa, EPI-589, NXN-462, Ferriprox, GM608, OXB-101, NTCELL, Ibiglustat, ENT-01, RG7935, and BIIB054.

132. The therapeutic drug according to claim 126, wherein the therapeutic drug for multiple sclerosis is at least one selected from the group consisting of steroid, interferon β, glatirameracetate, fingolimod, natalizumab, MN-166, siponimod, laquinimod, and masitinib.

133. A therapeutic or prophylactic drug for tauopathy or a dementia-related disease, comprising, as an active ingredient, a medicament capable of decreasing an amount of a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in the body of a patient with tauopathy or a dementia-related disease or a person possibly affected with tauopathy or a dementia-related disease, or a medicament capable of inhibiting the production of the polypeptide in the body of a patient with tauopathy or a dementia-related disease or a person possibly affected with tauopathy or a dementia-related disease, wherein
the tauopathy and dementia-related disease do not include Alzheimer's disease.

134. The drug according to claim 133, wherein the amount of the polypeptide is detected using an antibody.

135. A method for selecting a candidate substance for a therapeutic or prophylactic drug for tauopathy or a dementia-related disease, comprising using, as an index, reduction, by a test substance, of an amount of a polypeptide consisting of
(1) the amino acid sequence shown in SEQ ID NO: 1, or
(2) an amino acid sequence resulting from substitution, deletion, addition or insertion of one to several amino acids in the amino acid sequence shown in SEQ ID NO: 1,
in the body of a patient with tauopathy or a dementia-related disease or a person possibly affected with tauopathy or a dementia-related disease, or
inhibition, by a test substance, of the production of the polypeptide in the body of a patient with tauopathy or a dementia-related disease or a person possibly affected with tauopathy or a dementia-related disease, wherein the tauopathy and dementia-related disease do not include Alzheimer's disease.

136. The method according to claim 135, wherein a decrease in the amount of the polypeptide is detected using an antibody.
